(19) Europäisches Patentamt
European Patent Office
Office européen des brevets

(11) Publication number: 0 411 715 A2

# (12) EUROPEAN PATENT APPLICATION

(21) Application number: 90202113.8

(22) Date of filing: 02.08.90

(51) Int. Cl.5: C12N 15/57, C12N 15/62, C12N 15/74, C12N 1/21, C12N 9/52, A23L 1/226, //(C12N1/21,C12R1:225)

The title of the invention has been amended (Guidelines for Examination in the EPO, A-III, 7.3).

(30) Priority: 04.08.89 NL 8902010

(43) Date of publication of application: 06.02.91 Bulletin 91/06

(84) Designated Contracting States: GR

(71) Applicant: NEDERLANDS INSTITUUT VOOR ZUIVELONDERZOEK
Kernhemseweg 2 P.O. Box 20
NL-6710 BA Ede (Gld)(NL)

(72) Inventor: Vos, Petrus Antonius Josephina
Charles Crammweg 6
NL-6871 HZ Heelsum(NL)
Inventor: Siezen, Roelant Jan
Catharinadaal 19
NL-6715 KA Ede(NL)
Inventor: De Vos, Willem Meindert
Langhoven 50
NL-6721 SJ Bennekom(NL)
Inventor: Kok, Jan
Soerabajastraat 57a
NL-9715 LR Groningen(NL)
Inventor: Venema, Gerhardus
Kerklaan 26
NL-9751 NN Haren(NL)
Inventor: Haandrikman, Alfred Jacques
Albrondaheerd 24
NL-9737 RB Groningen(NL)

(74) Representative: de Bruijn, Leendert C. et al
Nederlandsch Octrooibureau
Scheveningseweg 82 P.O. Box 29720
NL-2502 LS 's-Gravenhage(NL)

## (54) Modified proteases, process for their preparation and their use in foodstuffs.

(57) The invention relates to DNA fragments containing at least one "mutant protease gene", which "mutant protease gene" is understood to mean:
- a gene made up of sections of parent protease genes of several lactococcal strains, in particular of the parent protease genes of L. lactis subsp. cremoris Wg2 and L. lactis subsp. cremoris SK11, as well as
- a parent protease gene of a lactococcal strain, in particular the parent protease gene of L.lactis subsp. cremoris Wg2 or the parent protease gene of L.lactis subsp. cremoris SK11, the DNA sequence of which has been altered in a manner such that in the mutant protease for which the gene codes:

(a) an amino acid other than that of the "wild-type" protease is present at at least one site, and/or
(b) at at least one site within the first 1350 residues of the amino acid sequence, calculated from the N-terminus, at least one amino acid of the "wild-type" protease is missing and/or one or more amino acids have been inserted, or
(c) at at least two sites separated from each other, one or more amino acids are missing and/or one or more amino acids have been inserted, the cloning vectors containing such a DNA fragment, the transformed host strains containing such a DNA fragment or vector, the mutant proteases obtainable as well as the foodstuffs and flavourings produced with the host strains or mutant proteases meant above.



Xerox Copy Centre

fig-3

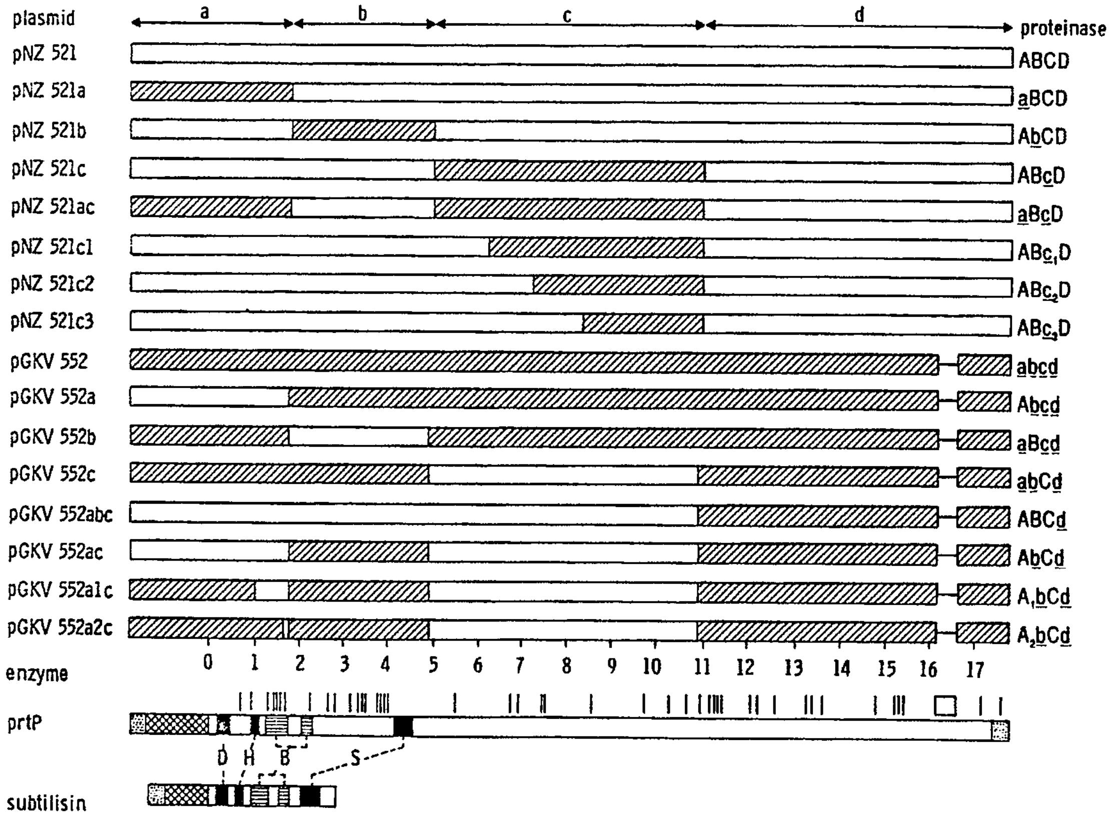
plasmid
a
b
c
d
proteinase
pNZ 521
ABCD
pNZ 521a
aBCD
pNZ 521b
AbCD
pNZ 521c
ABcD
pNZ 521ac
aBcD
pNZ 521c1
ABc1D
pNZ 521c2
ABc2D
pNZ 521c3
ABc3D
pGKV 552
abcd
pGKV 552a
Abcd
pGKV 552b
aBcd
pGKV 552c
abCd
pGKV 552abc
ABCd
pGKV 552ac
AbCd
pGKV 552a1c
A1bCd
pGKV 552a2c
A2bCd
enzyme
0 1 2 3 4 5 6 7 8 9 10 11 12 13 14 15 16 17
prtP
D H B S
subtilisin

# DNA FRAGMENT CONTAINING AT LEAST ONE PROTEASE GENE, A CLONING VECTOR CONTAINING SUCH A DNA FRAGMENT, A HOST CELL TRANSFORMED WITH A CLONING VECTOR CONSTRUCTED IN THIS MANNER, ALSO THE PROTEASES PRODUCED BY, OR PRODUCTS SUCH AS FOODSTUFFS PRE ARED WITH, SUCH HOST CELLS

The invention relates to a DNA fragment which contains at least one mutant protease gene.

As is known, gram-positive bacteria are used in the preparation of a large number of foodstuffs. Lactic acid bacteria are gram-positive bacteria which are used in the production of a series of fermented foodstuffs, including dairy products. These bacteria, in particular those which belong to the genus Lactococcus lactis (L.lactis), have a complex proteolytic system for degrading milk proteins to small peptides and free amino acids which are then used for the cell growth: see Thomas, T.D. et al., FEMS Microbiol. Rev. 46 (1987), pages 245-268. A key enzyme in this proteolytic system is a large serine-protease which is localized on the cell membrane and which is involved in the initial cleavage of caseins which are the most important milk proteins. This protease is essential for the cell growth and it produces peptides which contribute to the development of the flavour of a number of dairy products: see Visser, S. et al., Appl. Microbiol. Biotechnol. 29 (1988), pages 61-66. Complete secretion of the protease, normally bound to the outside of the cell membrane, can be brought about by changing the DNA coding for the C-terminal section of the protease; see de Vos, W.M., Biomolecular Engineering in the European Community (1986), pages 465-472, Martinus Nijhoff Publishers, The Netherlands. As a result of this secretion a simple isolation of the protease is possible on a large scale.

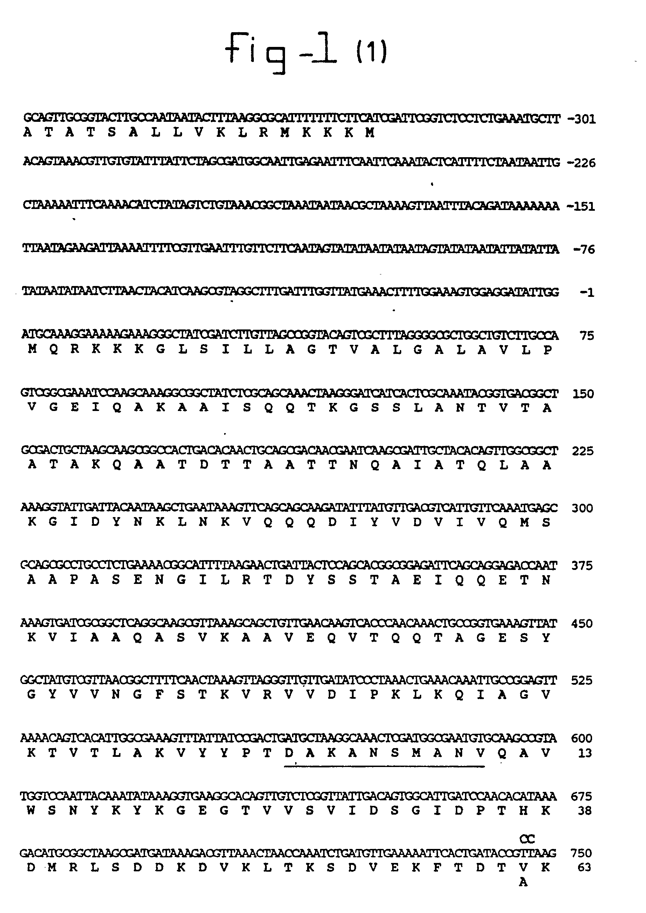

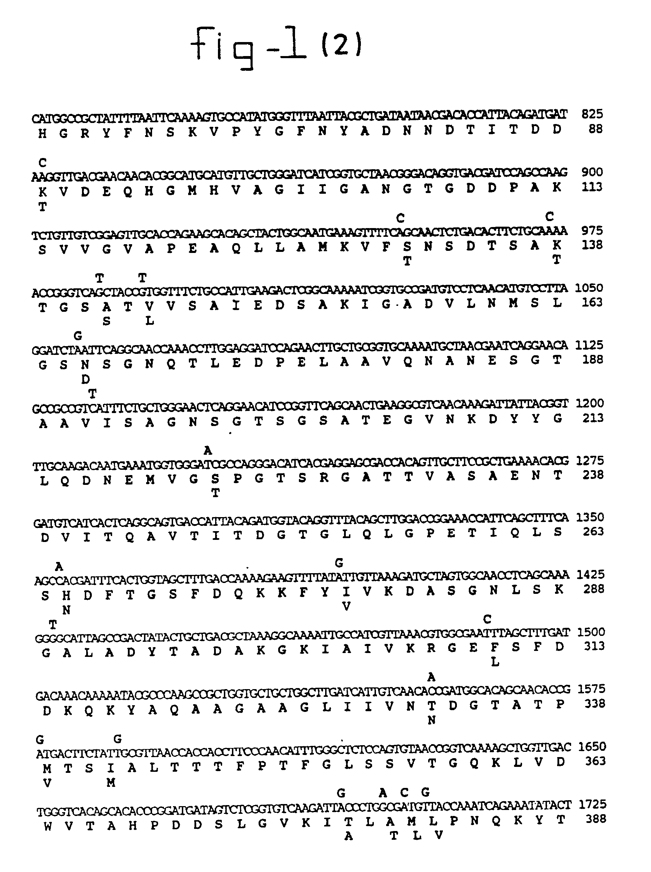

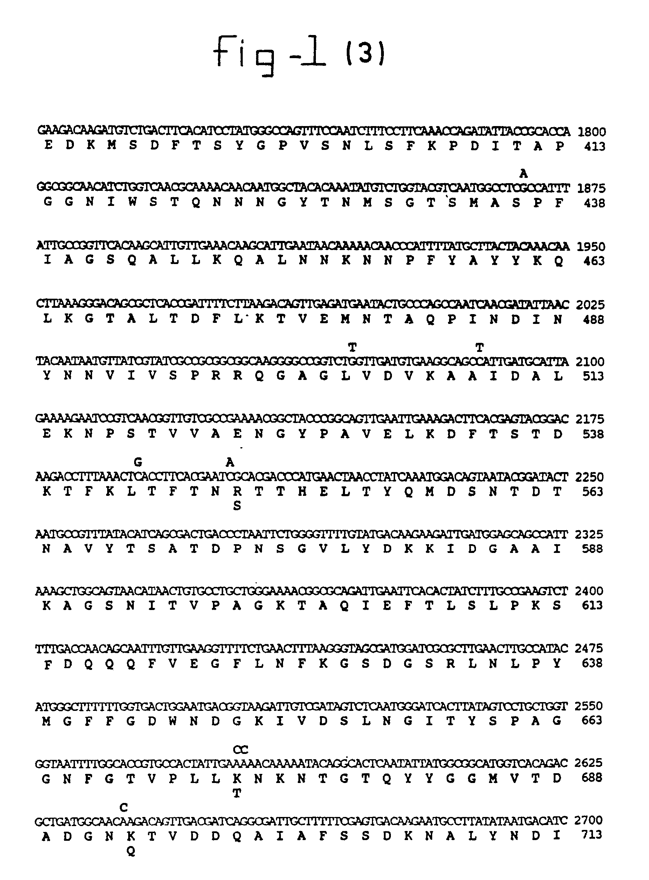

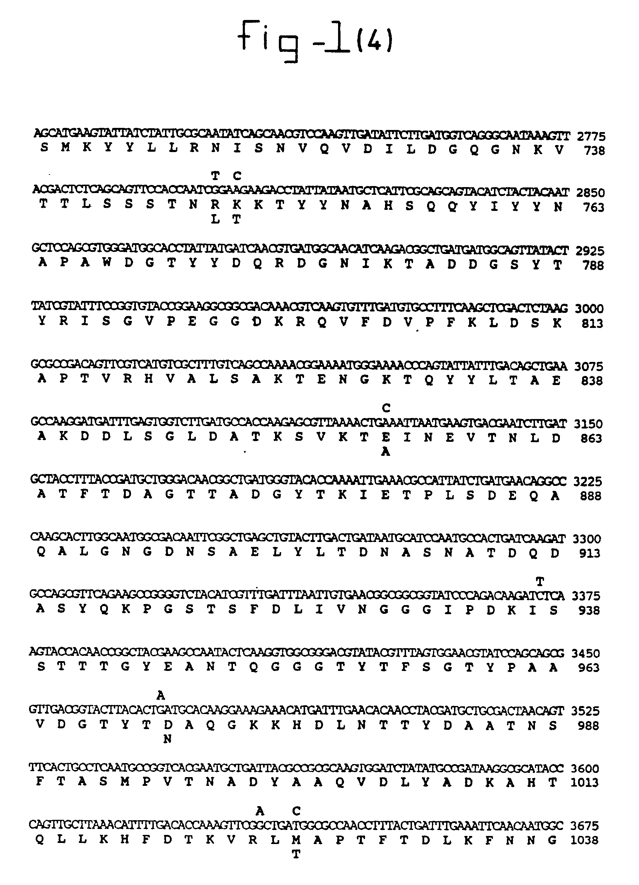

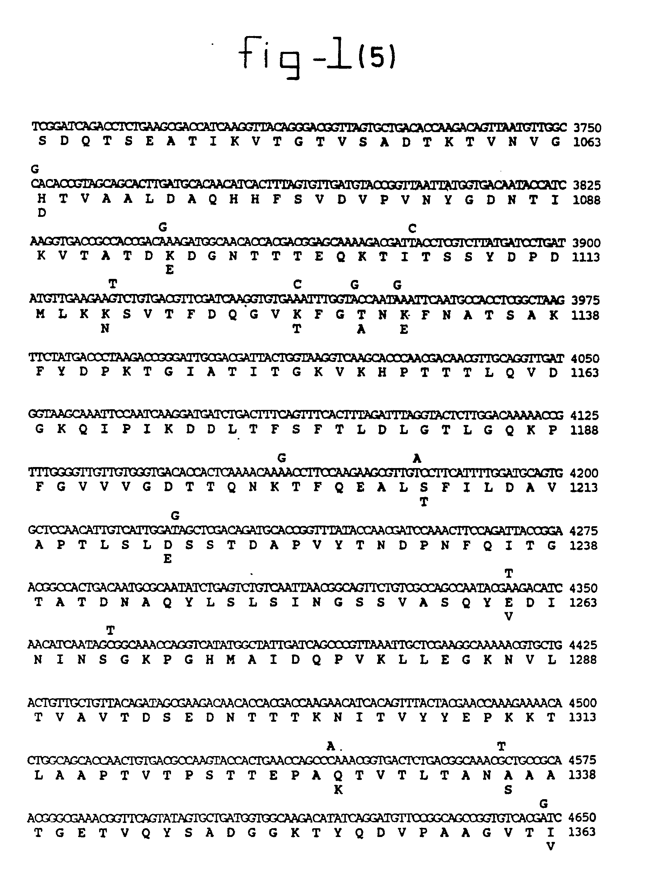

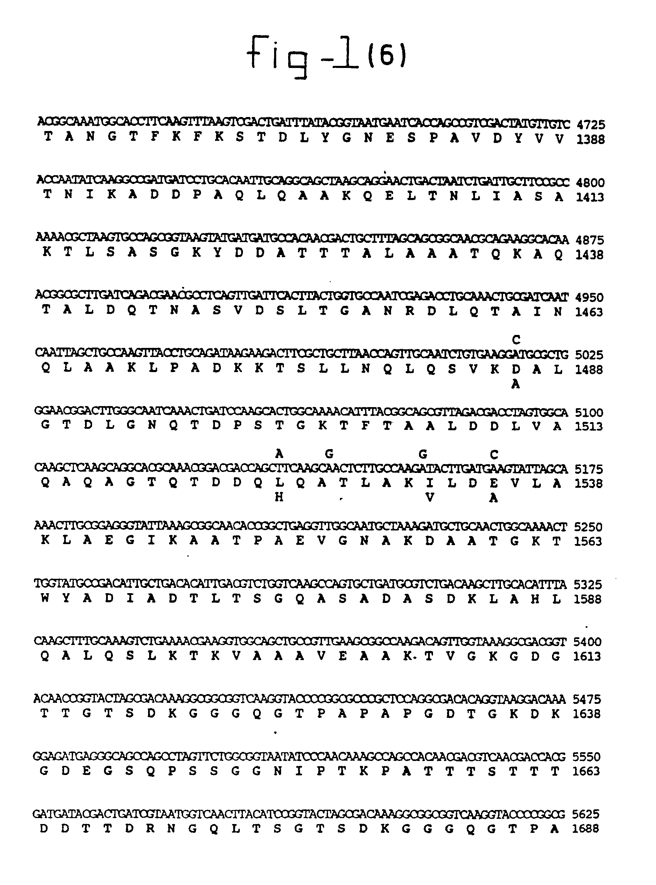

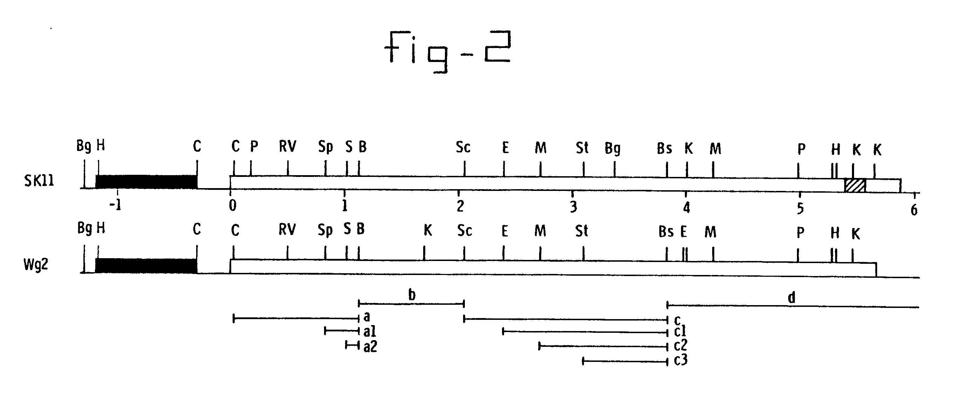

The proteases localized on the cell membrane can be divided into two main groups on the basis of their caseinolytic properties, viz. type I and type III proteases; see Visser, S. et al., Appl. Environ. Microbiol. 52 (1986), pages 1162-1166. Type III proteases degrade αs1-, β- and k-casein, while type I proteases mainly degrade β-casein. On acting on casein as a whole, type I proteases produce much more bitter-tasting peptides than type III proteases (see Visser, S. et al., Neth. Milk Dairy J. 37 (1983), pages 169-180). For the abovementioned two types of proteases it has been found that they are immunologically related proteins having similar molecular weights. The protease genes of a representative of each group, i.e. the type I protease of L.lactis Wg2 and the type III protease of L.lactis SK11 have been cloned and expressed in L.lactis ; see Kok, J., et al., Appl. Environ. Microbiol. 50 (1985) pages 94-101 and De Vos, W.M., Bimolecular Engineering in the European Community (1986), pages 465-472, Martinus Nijhoff Publishers, The Netherlands. In addition, the complete nucleotide sequence of the L.lactis Wg2 protease gene has been determined by Kok, J. et al., Appl. Environ. Microbiol (1988), pages 231-238 and the Applicant has determined the complete nucleotide sequence of the L.lactis SK11 protease gene (EP-A-0,307,011); these sequences are shown in Fig. 1. The protease genes code for very large pre-pro-proteins (>200 kDa) which are converted at the N- and C-terminus to obtain the mature active enzyme (Haandrikman, A. et al., J. Bacteriol. 171 (1989), pages 2789-2794 and Vos, P. et al., J. Bacteriol. (1989), pages 2795-2802). The amino acid sequence of the L.lactis SK11 and Wg2 proteases as derived from the nucleotide sequences are found to differ at 44 positions and furthermore the residues 1617-1676 are deleted in Wg2 protease in comparison with SK11 protease as shown in Table A (see also Kok, J. and Venema, G., Biochimie 70 (1988) pages 475-488). Furthermore, the lactococcal protease has an substantial sequence homology with a number of serine proteases of the subtilisin family; see Kok, J. et al., Appl. Environ. Microbiol. 54 (1988), pages 231-238. These homology regions are, inter alia, localized around the amino acid residues of the active site triad and in the substrate-binding region of the subtilisins, as indicated in Fig.3.

The Applicant has now carried out an investigation into a.o. the differences in the amino acid sequence between the L.lactis Wg2 and L.lactis SK11 proteases which should be responsible for the different cleavage specificity of the two enzymes. In doing this, starting from the genes which code for the proteases from L.lactis SK11 and Wg2 which are so-called "parent" proteases, new protease genes have been constructed which code for mutant proteases. In this investigation, mutant proteases have been developed in which "mutant protease gene" is understood to mean:

- a gene made up of sections of parent protease genes of several lactococcal strains, inter alia L.lactis SK11 and L.lactis Wg2, as well as
- a parent protease gene of a lactococcal strain, the DNA sequence of which has been altered in a manner such that, in the mutant protease for which the gene codes:

(a) an amino acid other than that of the "wild-type" parent protease is present at at least one site, and/or

(b) at at least one site within the first 1350 residues of the amino acid sequence, calculated from the N-terminus, at least one amino acid of the "wild-type" parent protease is missing and/or one or more amino acids have been inserted, or

(c) at at least two sites separated from one another, one or more amino acids of the "wild- type" parent protease are missing and/or one or more amino acids have been inserted.

The invention therefore relates to DNA fragments containing at least one mutant protease gene, the encoded protease having modified properties compared to the properties of the parent protease(s). These properties include the thermostability, alkaline/acid pH stability, oxidative stability, autoproteolysis, calcium ion binding, cleavage and substrate specificity, catalytic activity and pH activity profile of the proteases according to the invention. A particular embodiment of a mutant protease gene is a hybrid protease gene which is made up exclusively of sections of the Wg2 protease gene and the SK11 protease gene.

Moreover, the invention relates to cloning vectors, preferably as described in EP-A-0,157,441 or EP-A-0,228,726, which contain such a DNA fragment according to the invention, to host cells, advantageously lactococcal strains, in particular the L.lactis strain MG1363, deposited at the Centraal Bureau voor Schimmelcultures under number CBS 364.89, which have been transformed with a cloning vector specified above, to mutant proteases preparable with such host cells, and also to the products, such as foodstuffs and flavourings, which have been obtained with the aid of the host cells or proteases specified above.

The invention is illustrated in more detail by reference to the investigation described below.

A) The bacteria strains and plasmids used in the investigation

As host for transformation with M13 phages, use was made of E.coli TG1: K12, (Δ lac-pro), supE, thi, hsdD5/ F'traD36, $proA^{+}B^{+}$, $lacI^{q}$, lacZΔM15.

The strain L.lactis subsp. lactis MG1363 is a plasmid-free derivative of L.lactis subsp. lactis 712 and is described by Gasson, M.J., J. Bacteriol. 154 (1983), pages 1-9. This strain was used as host for all the plasmid transformations.

The plasmid pGKV552 contained the complete protease gene ( prt P gene) and a functional maturation protein gene ( prt M gene) of L.lactis subsp. cremoris Wg2, introduced into the lactococcal cloning vector pGK14, as described by Haandrikman, A. et al., J. Bacteriol. 171 (1989), pages 2789-2794. The prt M gene codes for a trans -active, maturation protein which, according to the Applicant, is regarded as essential for proteolytic activity. In this application the section of the plasmid which contains both the prt M gene and the prt P gene and also promoter regions for each is termed the protease region.

The plasmid pNZ521 contained the complete protease gene ( prt P gene) and a functional maturation protein gene ( prt M gene) of L.lactis subsp. cremoris SK11 introduced into the lactococcal cloning vector pNZ122, as described by Vos, P. et al. J. Bacteriol. 171 (1989), pages 2795-2802. Plasmid pNZ521 appears to have a reasonably high copy number in the strain L.lactis subsp. lactis MG1363 and, in addition, to be stably maintained without antibiotic selection pressure. The promoter region for the prt M and prt P genes of strain SK11 and Wg2 are not, completely identical. The prt M genes of strains SK11 and Wg2 code for identical maturation proteins prt M (Haandrikman A. c.s. and Vos P. c.s., J. Bacteriol. 171 , (1989), pages 2789-2802).

B) The molecular cloning method used in the investigation

The cloning method was carried out in accordance with generally known cloning techniques, such as those described by Maniatis, T., et al., Molecular Cloning: A Laboratory Manual, Cold Spring Harbor, New York, 1982, with only slight modifications. All the necessary enzymes were obtained from Bethesda Research Laboratories and New England Biolabs Corp. and were used in accordance with the supplier's instructions. The plasmids were introduced into the strain L.lactis MG1363 by means of electroporation (see Chassy, B.M. et al., FEMS Microbiol. Lett. 44 (1987), pages 173-177). The recombinant L.lactis strains were analysed by means of restriction enzyme analysis and in a number of cases by direct sequencing with double-strand plasmid DNA (see Tabor, S. et al., Proc. Natl. Acad. Sci. USA 82 (1987), pages 1074-1078 and Zhang, H. et al., Nucleic Acids Res. 16 (1988) page 1220) with the aid of specific oligonucleotide primers. These oligonucleotides were synthesized with the aid of a Biosearch Cyclone DNA synthesizer (New Brunswick Scientific Corp.).

C) Construction of the plasmids

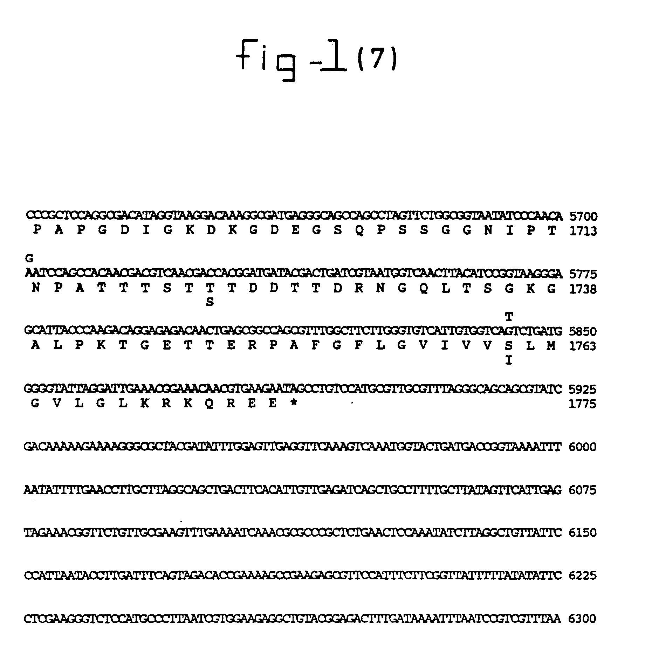

The plasmids containing the hybrid protease genes were constructed by replacing specific fragments of the L.lactis SK11 protease gene by the corresponding fragment of the L.lactis Wg2 protease gene and vice versa. The different fragments which have been exchanged are indicated in Fig. 2. In this connection, fragment (a) is a 1055 bp ClaI -BamHI fragment with the coding sequence of a section of the pre-pro-sequence and the first 173 amino acids of the mature protease. Fragment (b) is a 970 bp BamHI -SacII fragment which codes for the amino acids 174-496 and fragment (c) is a 1778 bp SacII -BstEII fragment which codes for the amino acids 497-1089 of the protease (see Fig. 2). In each of the fragments (a), (b) and (c), a restriction site is present which is unique to one of the two proteases, viz PstI , KpnI and BglII (see Fig. 2). The hybrid protease plasmids were therefore initially analysed with the aid of restriction enzyme analysis and then verified by a nucleotide sequence analysis at the relevant section of the plasmid DNA. As is shown in Fig. 3, the protease plasmids designated pNZ521a, pNZ521b and pNZ521c and also pGKV552a, pGKV552b and pGKV552c were constructed. Furthermore the fragment (c) of the SK11 protease was introduced into the plasmid pGKV552a, and this resulted in the plasmid pGKV552ac, and fragments (b) and (c) of the SK11 protease were introduced and this resulted in plasmid pGKV552abc. Furthermore the fragment (c) of the Wg2 protease was introduced in plasmid pNZ521a and this resulted in plasmid pNZ521ac. In addition, DNA fragments containing only sections of the fragment (c) of the Wg2 protease were exchanged in plasmid pNZ521; see Fig. 2. Fragment (c1) is a 1.45 kb EcoRI -BstEII fragment which corresponds to the amino acids 605-1089, fragment (c2) is a 1.11 kb MStI -BStEII fragment which corresponds to the amino acids 721-1089 and fragment (c3) is a 0.75 kb StyI -BStEII fragment which corresponds to the amino acids 840-1089. The plasmids obtained were designated pNZ521c1, pNZ521c2 and pNZ521c3. Furthermore DNA fragments containing only sections of fragment (a) of SK11 protease were introduced in plasmid pGKV552c; see Fig.2. Fragment (a1) is a 235 bp SphI -BamHI fragment which corresponds to to the amino acids 97-173 and fragment (a2) is a 33 bp SauI -BamHI fragment which corresponds to amino acids 163-173. The plasmids obtained were designated pGKV552a1c and pGKV552a2c. The used nomenclature for the hybrid proteases is as follows: wild-type SK11 and Wg2 proteases are designated ABCD and abcd respectively and hybrid proteases are indicated with the corresponding fragments of SK11 and Wg2 protease, which are present therein; see Fig. 3. The production of the functional protease, which was specified by each of these plasmids, was verified by analysis of the growth characteristics of L.lactis MG1363 strain containing these plasmids in milk.

D) Site-directed mutagenesis in the nucleotide sequence coding for the L.lactis cell wall protease

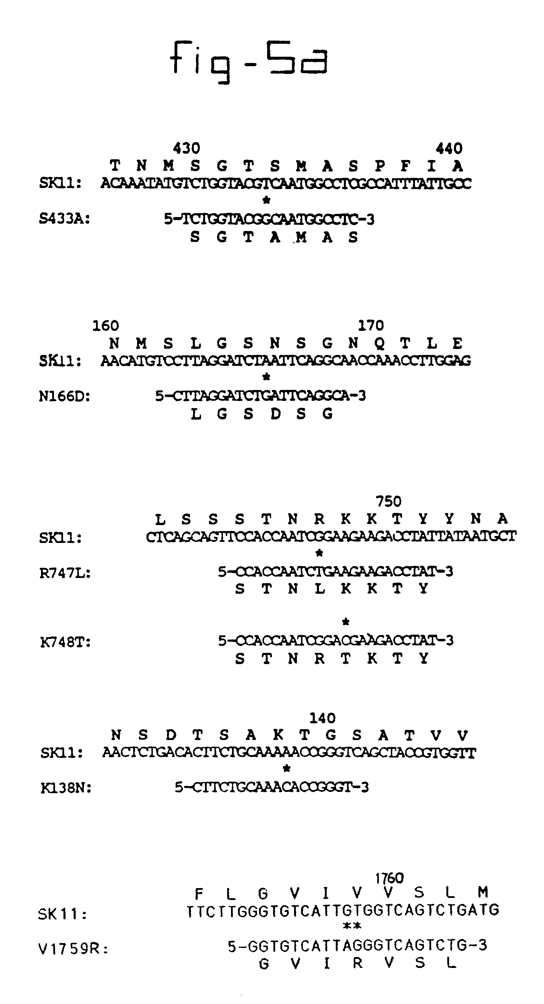

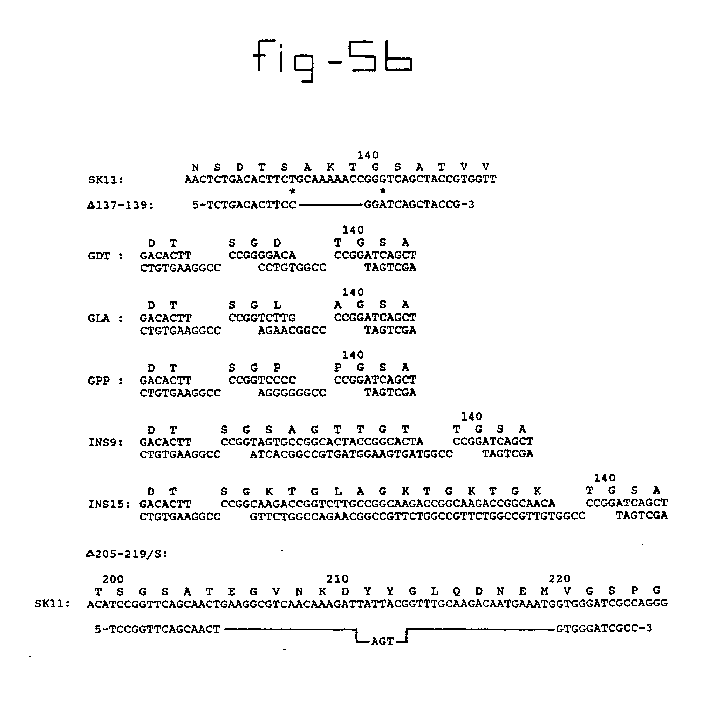

To make site-specific mutations in the protease gene, subfragments of the protease gene were first cloned in the cloning vector M13mp10 or M13mp11. Then the recombinant M13 phages obtained were isolated and single-strand DNA was isolated from them as described by Sanger, F. et al., Proc. Natl. Acad. Sci. USA 74 (1976), pages 5463-5467. Said single-strand DNA was hybridized with a complementary oligonucleotide which contained the desired mutation and said oligonucleotide was then used as a primer for synthesizing a complementary DNA strand as described by Zoller, M.J. and Smith, M., Nucleic Acids Res. 10 (1982), pages 6487-6500. Then the desired mutation was also introduced into the "template" DNA strand by the method due to Nakamaye, K. and Eckstein, F., which is described in Nucleic Acids Res. 14 (1986), pages 9679-9698. The double-strand DNA synthesized in this manner was transformed into E.coli strain TG1 and recombinant phages containing correct mutations were selected by determining the nucleotide sequence of the relevant section of the DNA of the M13 phage concerned, as described by Sanger, F. et al., Proc. Natl. Acad. Sci. USA 74 (1976), pages 5463-5467. As the final step, the subfragment of the protease gene containing the mutation made was replaced in the complete protease gene of L.lactis making use of cloning techniques as described in section B. An overview of the oligonucleotides used and the mutations in the SK11 protease gene resulting therefrom is shown in Fig. 5a. The obtained plasmids were designated (between the brackets the resulting mutation): pNZ574 (S443A), pNZ567 (K138N), pNZ573 (N166D), pNZ570 (R747L), pNZ571 (K748T) and pNZ590 (V1759R).

The result of one of the mutations made in the protease gene was a deletion of 9 nucleotides coding for amino acids A137, K138 and T139 of the L. lactis SK11 protease. The obtained plasmid was designated pNZ560. In addition, this produced a restriction site for the enzyme AccIII in the coding sequence at this position. This restriction site was then used to insert additional nucleotides at this position making use of synthetically-made 9 base pair double-strand adapter fragments. The result of some of these constructions is shown in fig. 5b. The obtained plasmids were designated (between brackets the resulting inserted amino acids): pNZ562 (GDT), pNZ563 (GLA), pNZ564 (GPP), pNZ565 (GSAGTTGTT) and pNZ566 (GKTGLAGKTGKTGKT). Furthermore a deletion mutation in the SK11 protease gene was made by means of a deletion of 45 nucleotides coding for amino acids E205-M219 followed by an insertion of three nucleotides coding for the amino acid serine. The plasmid obtained there from was designated pNZ576.

E) The isolation of the obtained mutant proteases

The protease was isolated by releasing it from the cells, in principle specifically in the manner described by Exterkate, F.A. et al., Appl. Environ. Microbiol. 49 (1985), pages 328-332. The L.lactis cells were cultured for 12-20 hours in a medium based on whey. Then the cells were collected by centrifuging and quickly washed once with "release" buffer (50 mM sodium acetate/ phosphate pH: 6.5). The cells were suspended in 1/50th of the original volume of the "release" buffer and incubated at 30° C while being shaken gently. The cells were again separated by centrifuging and the supernatant, which contained the protease released, was stored at 0° C. The cell pellet obtained on centrifuging was again suspended in "release" buffer and incubated at 30° C. Then the cells were again centrifuged, after which the two "release" fractions were combined and the pH was adjusted to a value of 5.4 with concentrated acetic acid. The protease isolated was placed in sample bottles and stored at -80° C.

F) Determination of the casein degradation

0, 5, 10 and 20 µl of each "release" fraction were incubated with 50 µg of αs1-, β- and k-casein in a total volume of 50 µl of 50 mM Na acetate/phosphate having a pH of 5.4 for 1 hour at 30° C. The reactions were terminated by adding an equal volume of a twice concentrated protein-gel sample buffer. 40µl of each sample was analysed on a 20% SDS-polyacrylamide gel.

The use of the methods and materials specified above is discussed in more detail below.

The construction of the L.lactis strains containing hybrid protease plasmids

As is evident from Fig. 2, the physical maps of the protease genes of L.lactis Wg2 and L.lactis SK11 are very similar. More particularly, the protease genes of both strains have a nucleotide sequence homology of 98.8%.

In view of the structure of hybrid protease genes, the plasmids pNZ521 and pGKV552 which both contain the structural protease gene ( prt P gene) and also the essential part of the prt M gene of L.lactis SK11 or L.lactis Wg2 were used. As stated, initially three different DNA fragments designated (a), (b) and (c) as shown in Fig. 2 were exchanged between the two protease plasmids and this resulted in the plasmids pNZ521a, pNZ521b, pNZ521c, pGKV552a, pGKV552b and pGKV552c. On the basis of the results obtained by the proteases produced by L.lactis strains containing such plasmids, a plurality of hybrid protease plasmids was constructed. Derivatives of pGKV552a were constructed viz. pGKV552ac and pGKV522abc which contain fragment (c) and both fragments (b) and (c) of the SK11 protease gene respectively. A derivative of pNZ521a was constructed which also contained fragment (c) of the Wg2 protease gene and was designated pNZ521ac. Furthermore, derivatives of the plasmid pNZ521c were constructed which contained only a section of the fragment (c) of the Wg2 protease gene, and this resulted in the plasmids pNZ521c1, pNZ521c2 and pNZ521c3 and derivatives of plasmid pGKV552C were constructed which only contained a section of fragment (a) of the SK11 protease gene and resulted in the plasmids pGKV552a1C and pGKV552a2c (see Fig. 2). A diagrammatic representation of the proteases which have been synthesized by the L.lactis strains containing the plasmids concerned is shown in Fig. 3.

The cleavage specificity of the hybrid Wg2/SK11 proteases according to the invention

The protease was isolated from the L.lactis strains by releasing the enzyme from the cell membrane by incubation in a $Ca^{2+}$-free buffer. To determine the cleavage specificity of the proteases concerned, various quantities of all the separate protease preparations were incubated with αs1-, β- and k-casein and the casein degradation products obtained were separated on SDS-PAGE gels.

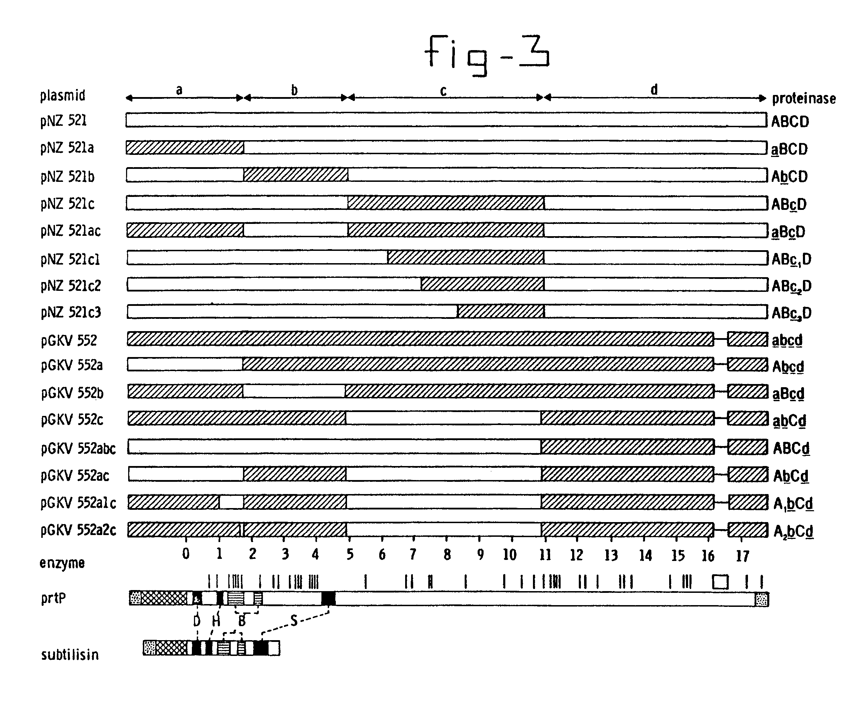

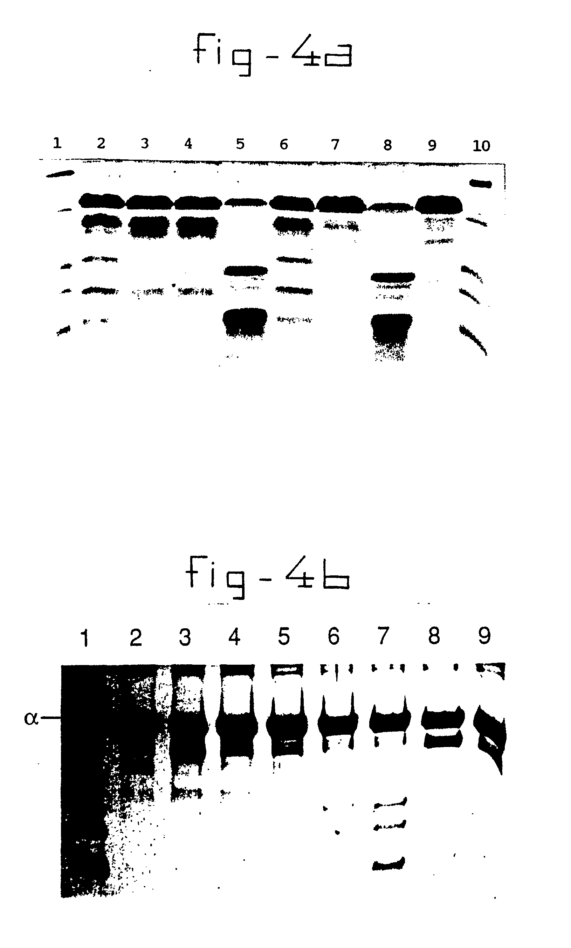

It is generally known that the Wg2 protease degrades αs1-casein very inefficiently and this is in contrast to the protease in strain SK11. This is borne out by the results which have been obtained with the proteases which have been specified by the plasmids pGKV552 (Wg2) and pNZ521 (SK11) (see Fig. 4a and 4b). It has been found that hybrid proteases which contain only fragment (a) or (c) of SK11 protease are in fact able to degrade αs1-casein but not nearly so efficiently as, and with a different cleavage specificity from, the "wild type" SK11 protease (Fig. 4a and 4b). An exchange of the fragment (b) and/or fragment (d) does not affect the cleavage specificity for αs1-casein. More particularly it is supposed that the protease which is specified by the plasmid pGKV552ac is the only hybrid derived from the Wg2 protease which is capable of efficiently cleaving the αs1-casein, comparable to the wild-type SK11 protease. The abovementioned results indicate that at least one of the fragments (a) and (c) of the SK11 protease is necessary to degrade the αs1 casein and both fragments are necessary to efficiently degrade the αs1-casein. It is, however, very remarkable and particularly unexpected that fragment (c) of the SK11 protease is necessary to degrade αs1-casein since fragment (c) does not have any homology with subtilisin or other serine proteases which are in fact capable of degrading αs1-casein.

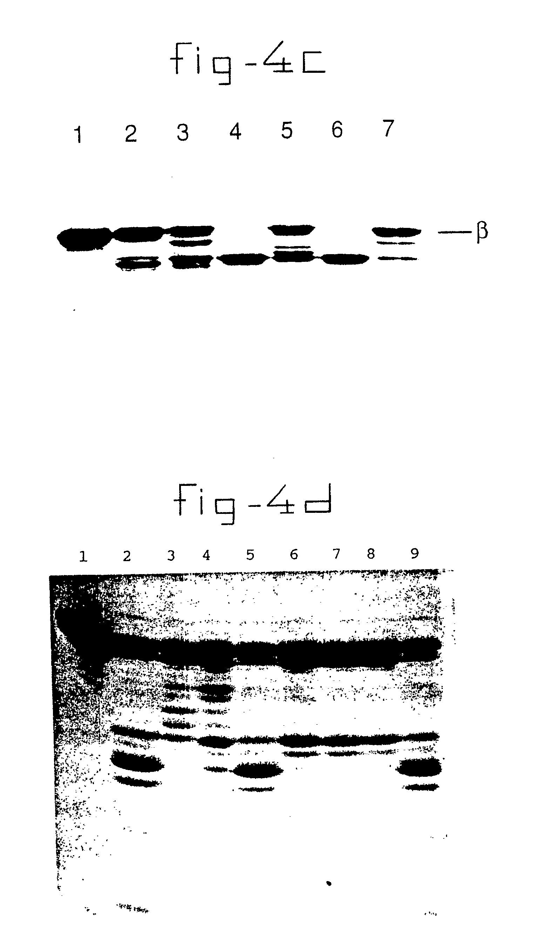

As is generally known the L.lactis SK11 and Wg2 proteases are both capable of degrading β-casein, but with a markedly different specificity (see Visser, S. et al., Appl. Environ. Microbiol. 52 (1986), pages 1162-1166). In this connection it is pointed out that the various hybrid proteases according to the invention were also capable of degrading β-casein. However, the fragments (a) and (c) both altered the cleavage specificity for β-casein (Figs. 4c and 4d). These findings bear out the results of the cleavage specificity of the hybrid proteases for αs1-casein. Fragments (a) and (c) both affect the cleavage specificity of Lactococcus protease for β-casein, and fragment (b) has no effect. The striking fact is that L.lactis MG1363 transformed with either pNZ521a or with pGKV552c yields hybrid proteases which both appear to have one and the same cleavage specificity. The same is true of L.lactis MG1363 transformed with either pNZ521c or with pGKV552a (Fig. 4c). It also appears that L.lactis MG1363 bacteria transformed with either pGKV552 or pGKV552b have an identical cleavage specificity. The same is true of L.lactis bacteria transformed with pGKV552ac, pNZ521, pNZ521b or pNZ521c3 which all degrade β-casein in the same manner. These findings indicate that fragment (a) and fragment (c) are essential for determining the specificity and that, in the case of the hybrid proteases investigated, it is not important whether the other sections of the protein originate from strain SK11 or Wg2. A combination of fragment (a) of the one protease with fragment (c) of the other protease results in a hybrid protease having a specificity which differs from both SK11 and Wg2 proteases.

In connection with the above it is emphasized that it was possible to ascribe a large portion of the degradation products obtained with the hybrid proteases to SK11-type or Wg2-type activities, but some of the degradation products were not observed in the case of the "wild type" enzymes. Furthermore, bands having a similar mobility were produced in different amounts, with the result that it is likely that the affinity for some cleavage sites in the casein and/or the kinetics of the degradation varied appreciably between the different proteases. In addition it is noted that fragment (a) corresponds to a sequence of 173 amino acids in which only 7 amino acid substitutions between the SK11 protease and Wg2 protease have been found (see Table A). The degradation of αs1-casein by the proteases specified by the plasmids pGKV552a1c and pGKV552a2c (see Fig. 3) was investigated for verifying the influence of these substitutions on the cleavage specificity. The obtained results indicated that one or more of the amino acid substitutions at the positions 131, 138, 142 and 144 are the most important substitutions in this respect as the protease specified by the plasmids pGKV552a and pGKV552a1c on the one hand and the proteases specified by the plasmids pGKV552c and pGKV552a2c on the other hand produce almost identical cleavage patterns of αs1-casein (see Fig.4b).

Furthermore, it is emphasized that fragment (c) corresponds to a region of 593 amino acids in which only 9 amino acid substitutions have been found between the SK11 and Wg2 proteases (see Table A). The β-casein degradation by the proteases specified by the plasmids pNZ521c1, c2 and c3 (see Fig. 3) was investigated in order to determine which of these substitutions affects the cleavage specificity. The results obtained indicate that the amino acid substitutions at positions 747 and 748 (see Table A) are very important in this connection since the proteases specified by the plasmids pNZ521c3 and pNZ521, on the one hand, and the proteases specified by the plasmids pNZ521c, pNZ521c1 and pNZ521c2, on the other hand, produce identical cleavage patterns of β-casein (see Fig. 4d). The section of the fragment (c) which is involved in the substrate binding is therefore very probably localized around amino acids 747 and 748.

**TABLE A**

Amino acids exchanged in the various hybrid proteases

| a.a.[a] | SK11[b] | Wg2[b] | fragment[c] |
|---|---|---|---|
| 62 | V | A | a |
| 89 | K | T | a |
| 131 | S | T | a,a1 |
| 138 | K | T | a,a1 |
| 142 | A | S | a,a1 |
| 144 | V | L | a,a1 |
| 166 | N | D | a,a1,a2 |
| 222 | S | T | b |
| 265 | H | N | b |
| 278 | I | V | b |
| 310 | F | L | b |
| 332 | T | N | b |
| 339 | M | V | b |
| 342 | I | M | b |
| 378 | T | A | b |
| 380 | A | T | b |
| 381 | M | L | b |
| 382 | L | V | b |
| 548 | R | S | c |
| 673 | K | T | c,c1 |
| 693 | K | Q | c,c1 |
| 747 | R | L | c,c1,c2 |
| 748 | K | T | c,c1,c2 |
| 855 | E | A | c,c1,c2,c3 |
| 970 | D | N | c,c1,c2,c3 |
| 1026 | M | T | c,c1,c2,c3 |
| 1064 | H | D | c,c1,c2,c3 |
| 1095 | K | E | d |
| 1117 | K | N | d |
| 1126 | K | T | d |
| 1129 | T | A | d |
| 1131 | K | E | d |
| 1207 | S | T | d |
| 1220 | D | E | d |
| 1261 | E | V | d |
| 1328 | Q | K | d |
| 1336 | A | S | d |
| 1363 | I | V | d |
| 1486 | D | A | d |
| 1525 | L | H | d |
| 1532 | I | V | d |
| 1535 | E | A | d |
| 1617-1676 | +(d) | -(d) | |
| 1722 | T | S | d |
| 1761 | S | I | d |

a) Amino acid position of the specific mutation.
b) Type of amino acid at the specified position of the SK11 and Wg2 protease. The single-letter code is used for amino acids (see Table B).
c) DNA fragment which contains the specified amino acid.
d) segment present (+) or not present (-)

The cleavage specificity of the different proteases for k-casein was also investigated. In this case, similar results were obtained as for the degradation of β-casein: both fragments (a) and (c) are important for the cleavage specificity. Here again it holds true that some hybrid proteases have different caseinolytic properties compared with those of the "wild-type" enzymes. This is characterized by

(i) a different rate and/or efficiency of cleavage of peptide bonds which were also recognized by the "wild-type" enzymes, and/or

(ii) the cleavage of peptide bonds which were not detected in the case of the "wild-type" enzymes.

In addition to the degradation of caseins, the cleavage specificity of the various hybrid and original proteases towards other proteins was also investigated. In this case, similar results were obtained as for the degradation of caseins.

Furthermore, the Applicant has found in fragment (a) amino acid sequences which have a homology with the substrate binding region of serine proteases of the subtilisin family. In this connection, the Applicant assumes that a similar role occurs for this region in the lactococcal proteases. However, the discovery that fragment (c) also affects the cleavage specificity is surprising and makes it probable that, compared with the subtilisins, the lactococcal proteases contain additional elements for determining the cleavage specificity. The proteases described here therefore have two regions which are involved in the binding and cleavage of substrate molecules. One region is situated in fragment (a) after residue 130 and is homologous with the substrate binding region of serine proteases of the subtilisin family (Wells., J. et al., Proc. Natl. Acad. Sci. USA 84 (1987), pages 5167-5171; McPhalen, C.A., et al., Proc. Natl. Acad. Sci. USA 82 (1985), pages 7242-7246; Betzel, C. et al., Eur. J. Biochem. 178 (1988), pages 155-171). The other region is situated at residues 747 and 748 in fragment (c); a corresponding (homologous) region is not, however, present in subtilisins or other serine proteases.

## Mutant proteases obtained by site-directed mutagenesis

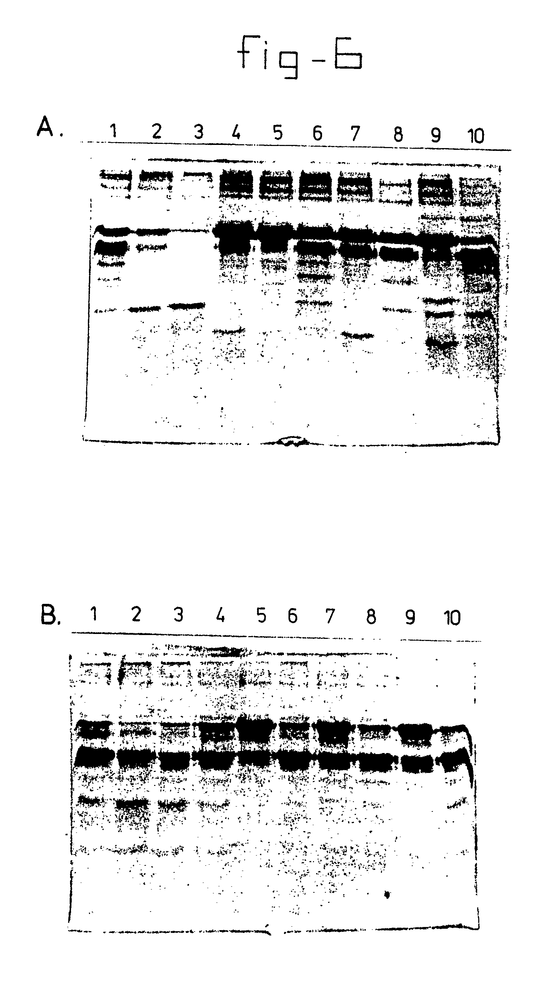

Furthermore, mutant proteases having new cleavage specificities can also be obtained by carrying out single amino acid substitutions. These substitutions then have to be carried out, in particular, in the regions which are essential for the cleavage specificity. Thus, amino acid serine 433 of L.lactis SK11 protease was replaced by alanine, as a result of which the so-called S433A mutant SK11 protease was obtained (Fig. 5a). This S433A mutant proved to be completely inactive, which bears out the hypothesis that S433 represents the serine residue of the active site triad. Single amino acid substitutions were also made at positions 138 and 166 of L.lactis SK11 protease, as a result of which, respectively, the K138N and N166D mutant proteases were obtained (Fig. 5a). Both the K138N and the N166D mutant proteases appeared to cleave αs1 and/or β-casein in a different manner from "wild-type" SK11 or Wg2 protease (Fig. 6a and 6b). This demonstrates that the amino acid residues at positions 138 and 166 situated on fragment (a) (see Table A) both affect the cleavage specificity. Single amino acid substitutions have also been made at positions 747 and 748 of L.lactis SK11 protease, both situated on fragment (c) (see Table A) and of importance for the cleavage specificity, as a result of which the R747L and K748T mutant proteases have been obtained (Fig. 5a). The K748T mutant protease appeared to be as stable as the wild type SK11 protease with respect to autoproteolysis whereas the R747L mutant protease appeared to be much more sensitive to autoproteolysis. This last aspect could be the consequence of a strongly enhanced proteolytic activity and/or a much broader specificity. The enzyme was not stable enough for performing digestions of α- and β-casein. On the other hand the more stable K748T mutant protease appeared to degrade αs1-casein in a manner clearly different from the wild type SK11 protease, which confirms that amino acid 748 does have an influence on the cleaving specificity and/or activity, respectively.

Furthermore, mutant proteases having altered properties can be obtained by omitting (so-called "deletion") or introducing (so-called "insertion") one or more amino acids. If this deletion or insertion takes place in a region which is essential for the cleavage specificity, mutant proteases can be obtained having novel catalytic properties. Thus, a mutant protease derived from L.lactis SK11 protease was made in which amino acids A137, K138 and T139 are missing (Fig. 5b). This includes a residue K138 which has been found to govern the cleavage specificity. This Δ137-139 mutant SK11 protease was also found to cleave αs1- and β-casein in a different manner from "wild-type" SK11 or Wg2 protease (Fig. 6a and 6b). In the construction of this mutant protease gene, a new AccIII restriction enzyme cleavage site was also introduced into the DNA fragment, as a result of which it became possible to insert one or more codons at the site where the codons for residues 137-139 have been omitted. Thus, inter alia, mutant proteases derived from SK11 proteases were made, the residues 137-139 (i.e. AKT) being replaced by the three residues GDT, GPP and GLA, resulting in the mutant proteases A137G/K138D, A137G/K138P/T139P and A137G/K138L/T139A respectively. Furthermore mutant proteases, termed Δ137-139/ins9 and Δ137-139/ins15, were made, the residues 137-139 (i.e. AKT) being replaced by 9 residues (i.e. GSAGTTGTT) and fifteen residues (i . e. GKTGLAGKTGKTGKT), respectively (Fig. 5b). All these mutant proteases obtained by insertion of 3-15 amino acids at positions 137-139 appeared to be stable and active and they appeared to cleave αs1-casein and β-casein in a manner different from wild type SK11 protease or wild type Wg2 protease (Fig. 6a and 6b).

Amino acid deletions or insertions can also be made in regions which determine properties of the protease other than cleavage specificity. Thus, a mutant protease derived from SK11 protease, termed Δ205-219/S, was made in which the segment containing amino acids E205-M219 was replaced by a single serine residue (Fig. 5b). This omitted segment contains, in particular, peptide bonds which were found to be sensitive to autoproteolysis, as a result of which the "wild-type" SK11 protease may inactivate itself. As a result of the deletion of this segment in the said Δ205-219/5 mutant, these cleavage-sensitive peptide bonds are no longer present.

The C-terminal section of L.lactis serine protease is responsible for binding to the cell membrane. By deleting the C-terminal section of the SK11 proteases from residue 1373 ( SalI restriction site) it is possible to obtain an active mutant protease which is completely secreted into the medium (Vos, et al., J. Bacteriol. 171 (1989), pages 2795-2802). According to the present invention it has been determined what the effect is of a mutation in the hydrophobic area of the C-terminal section which is completely or partially responsible for the anchoring in the cell membrane. For this purpose the mutation V1759R was introduced in the L.lactis SK11 protease, as a result of which the mutant V1759R protease was secreted almost completely into the medium. In addition, it has been determined according to the invention which effect a further shortening of the C terminus has on the activity and stability of the shortened secreted SK11 protease. In this connection it was found that curtailment to 1272 amino acids ( NdeI restriction site) still in fact yields an active and stable secreted protease, while curtailment to 1090 amino acids ( BstEII site in Fig. 2) or 937 amino acids ( BglII site in Fig. 2) no longer yielded an active and/or stable protease. This demonstrates that between 1090 and 1272 amino acids calculated from the mature N-terminus are minimally necessary to produce an active and/or stable mutant protease.

With reference to the above, the invention relates, in particular, to DNA fragments which contain at least one mutant protease gene, which gene is made up of sections of protease genes of several lactococcal strains or of a protease gene of one lactococcal strain, the DNA sequence of which has been altered at one or more sites. Advantageously, such a mutant protease gene is made up of at least sections of a protease gene, in particular a type I protease gene of L.lactis , more particularly of L.lactis subsp. cremoris Wg2 and a protease gene, in particular a type III protease gene of L.lactis , more particularly of L.lactis subsp. cremoris SK11.

The invention also relates to DNA fragments which contain at least one hybrid protease gene which is made up of the type I protease gene or L.lactis subsp. cremoris Wg2, of which one or more codons coding for amino acids as shown in Table A, in particular the amino acids A62, T89, T131, T138, S142, L144, D166, L747 and T748, have been altered into codons which code for the amino acids specified in Table A at corresponding sites in the type III protease gene of L.lactis subsp. cremoris SK11. In addition to these last-mentioned DNA fragments, the invention also relates to DNA fragments containing at least one hybrid protease gene which is made up of the type III protease gene of L.lactis subsp. cremoris SK11, of which one or more codons coding for amino acids as shown in Table A, in particular the amino acids V62, K89, S131, K138, A142, V144, N166, R747 and K748, have been altered intc codons which code for the amino acids specified in Table A, at corresponding sites in the type I protease gene of L.lactis subsp cremoris Wg2.

Furthermore, the invention relates to DNA fragments which contain at least one mutant protease gene which is made up of the type I protease gene of L.lactis subsp. cremoris Wg2, of which one or more codons coding for the amino acids as shown in Table A, in particular the amino acids A62, T89, T131, T138, S142, L144, D166, L747 and/or T748, have been altered into codons which do not code for the amino acids specified in Table A at corresponding sites in the type III protease gene L.lactis subsp. cremoris SK11. In addition, the invention relates to DNA fragments which contain at least one mutant protease gene which is made up of the type III protease gene of L.lactis subsp. cremoris SK11, of which one or more codons coding for amino acids as shown in Table A, in particular the amino acids, v62, K89, S131, K138, A142, V144, N166, R747 and K748, have been changed into codons which do not code for the amino acids specified in Table A at corresponding sites in the type I protease gene of L.lactis subsp. cremoris Wg2.

The invention also relates to DNA fragments which contain at least one mutant protease gene which is made up of the type I protease gene of L.lactis subsp. cremoris Wg2, of which one or more codons are missing, including, preferably, at least one codon coding for the amino acids A62, T89, T131, T138, S142, L144, D166, L747, and/or T748, and/or to which one or more codons have been added. The invention also relates to DNA fragments which contain at least one mutant protease gene which is made up of the type III protease gene of L.lactis subsp cremoris SK11, of which one or more codons are missing, including, preferably, at least one codon coding for the amino acids V62, K89, S131, K138, A142, V144, N166, R747 and/or K748, and/or to which one or more codons have been added.

As already stated, the invention also relates to the cloning vectors, including plasmids, which contain a DNA fragment according to the invention, to host cells, preferably lactococcal strains, in particular the L.lactis strain MG1363, which have been transformed with a cloning vector specified above, to the proteases obtained with such host cells and also to the foodstuffs, flavourings etc., which are obtained with the aid of said host cells or proteases. With regard to this last aspect, it is emphasized that, although the invention has been primarily illustrated by the preparation of modified genes, the expression of them in lactic acid bacteria and the cleavage of caseins with the modified proteases is the object of the investigation which has led to the invention. The invention is therefore based on providing new proteases and cultures containing these proteases for preparing all kinds of products which can be prepared with the aid of lactic acid bacteria. The protein cleavage products formed in this manner are either used as such or further reacted. Examples of such products are butter, margarines and other spreads for bread which contain fermented milk or skimmed milk in the aqueous phase. Furthermore, other fermented milk products such as butter milk and fermented skimmed milk, quark and other fresh and ripened types of cheeses and yoghurt and also flavouring mixtures are obtained by making use of the modified proteases according to the invention, while human or animal foodstuffs based on proteins other than milk proteins can also be prepared with the aid of the proteases according to the invention.

An important embodiment of the invention is therefore the use of one or more of the mutant proteases described above or of cultures which contain one or more of said mutant proteases for preparing the products just specified which contain protein cleavage products.

## LEGENDA OF THE FIGURES

### Fig. 1: Nucleotide sequence of the L. lactis SK11 protease gene and the amino acid sequence of the protease derived therefrom.

The numberings of the nucleotides and amino acids are indicated: nucleotide position 1 is the first base of the start codon of the protease gene, while amino acid position 1 is the first amino acid (D) of the mature protease. In addition, the first 10 amino acids of the mature protease are underlined. The first 17 amino acids of the prt M protein which is coded upstream of the prt P gene in reverse order, starting with M, K, K, K, M, R etc., are also indicated. The differences in the nucleotide sequence from the protease gene of L.lactis Wg2 are indicated above each line, while the differences in the amino acid sequence from the protease of L.lactis Wg2 are indicated under each line. The single-letter code for amino acids is used (see Table B).

### Fig. 2: Structure of hybrid protease plasmids.

The upper and lower diagrams from the top show the protease regions on plasmids pSK111 and pWVO5 of, respectively, strain SK11 and Wg2, with the prt M gene on the left and the prt P gene on the right. The blank bars indicate the coding regions of the prt P gene and the black bars indicate the coding regions of the prt M gene. The hatched bar indicates the duplication of 180 bp which is present in the SK11 prt P gen. The lengths are indicated in kb, position 0 being the initiation codon of the protease gene. The cleavage sites of each relevant restriction enzyme are indicated by: B, BamHI ; Bg, BglII ; Bs, BstEII ; C, ClaI ; E, EcoRI , H, HindIII ; K, KpnI ; M, MstI ; P, PstI ; RV, EcoRV ; S, SauI ; Sc; SacII ; Sp, Sph I; St, StyI (not all Sty I-sites are indicated).

The various DNA fragments which have been exchanged between the two protease genes are shown at the bottom.

### Fig. 3: Diagrammatic representation of various hybrid proteases.

The nature of the protease gene which is present in the various plasmids is shown, with the plasmid code indicated on the left and the protease code indicated on the right, the sequences derived from SK11 being shown as blank bars and the sequences derived from Wg2 as hatched bars. The "wild-type" SK11 and Wg2 protease genes are present on plasmids pNZ521 and pGKV552, respectively. At the top the DNA fragments are shown which were exchanged between both protease genes (see Fig.2). The sizes of the mature protease coded by these plasmids are indicated in units of 100 amino acids. The proteases and subtilisin are shown diagrammatically at the bottom of the figure, with the pre-peptide (on the left) and the membrane anchor (on the right) dotted, the pro-peptide cross-hatched and the most homologous regions in black, which are around the active site amino acid residues aspartic acid (D), histidine (H) and serine (S). The horizontal hatched regions, further indicated with B, represent the substrate binding region in subtilisin and the corresponding area in the L.lactis protease. The vertical strokes above the lactococcal protease indicate approximately the position of the amino acid differences between the proteases of strain SK11 and strain Wg2 (see Table A). The blank square indicates the position of the deletion of 60 amino acids in Wg2 protease in comparison with SK11 protease.

**Fig. 4a: SDS polyacrylamide gel patterns of αs1-casein incubated with release fractions of "wild-type" and hybrid protease-containing L. lactis strains**

Lanes 1 and 10 contain marker proteins of ovalbumin, carbonic anhydrase, β-lactoglobulin, lysozyme and bovine trypsin inhibitor having molecular weights of, respectively, 43, 29, 18.4, 14.3 and 6.2 kilodaltons. Lane 9 contains αs1-casein not incubated with release fraction. Lanes 2 to 8 inclusive contain αs1-casein incubated with release fractions of L.lactis MG1363 containing, respectively, plasmids pGKV552c, pGKV552a, pNZ521c, pNZ521b, pNZ521a, pGKV552 and pNZ521.

**Fig. 4b: SDS-polyacrylamide gel patterns of αs1-casein incubated with release fractions of "wild-type" and hybrid protease containing L.lactis strains.**

Lanes 1 to 9 contain αs1-casein incubated with release fractions of L.lactis MG1363 containing, respectively, plasmids pNZ521, pGKV552, pGKV552a, pGKV552b, pGKV552c, pGKV552ac, pGKV552abc, pGKV552a1c and pGKV552a2c.

**Fig. 4c: SDS-polyacrylamide gel patterns of β-casein incubated with release fractions of "wild-type" and hybrid protease-containing L.lactis strains.**

Lane 1 contains β-casein not incubated with a release fraction. Lanes 2 to 7 contain B-casein incubated with release fractions of L.lactis MG1363 containing, respectively, plasmids pNZ521, pNZ521a, pNZ521c, pGKV552, pGKV552a and pGKV552c.

**Fig. 4d: SDS-polyacrylamide gel patterns of β-casein incubated with release fractions of "wild-type" and hybrid protease-containing L.lactis strains.**

Lane 1 contains β-casein incubated with release fraction of the plasmid-free, protease-deficient strain L.lactis MG1363. Lanes 2 to 9 inclusive contain β-casein incubated with the release fractions of L.lactis MG1363 containing, respectively, the plasmids pNZ521, pGKV552, pNZ521a, pNZ521b, pNZ521c, pNZ521c1, pNZ521c2 and pNZ521c3.

**Figs. 5a and 5b: Overview of the oligonucleotides used in the site-directed mutagenesis of the L.lactis SK11 protease gene.**

The "wild-type" L.lactis SK11 nucleotide sequence is in each case indicated in the top line with the amino sequence above it and its position in the protease. The nucleotide sequence of the oligonucleotide used and the amino acid sequence resulting therefrom is in each case shown underneath. The altered nucleotides are indicated by *. In the case of the construction of deletion mutants, the two nucleotides on either side of the deletion concerned are connected by a line. In the case of the construction of the insertion mutants, the inserted double-strand oligonucleotide concerned and the altered amino acid sequence resulting therefrom are shown. The nomenclature of mutants is given at the left.

**Fig. 6a: SDS-polyacrylamide gel patterns of αs1-casein incubated with release fractions of "wild-**

**type" and mutant protease containing L.lactis strains.**

Lanes 1 to 10 contain αs1-casein incubated with release fractions of L.lactis MG1363 containing respectively plasmids pNZ567 (K138N), pNZ573 (N166D), pNZ521 (wild. type), pNZ562 (GDT), pNZ564 (GPP), pNZ563 (GLA), pNZ560 (Δ137-139), pNZ571 (K748T), pNZ565 (ins9) and pNZ566 (ins15).

**Fig. 6b: SDS-polyacrylamide gel patterns of β-casein incubated with release fractions of "wild-type" and mutant protease containing L.lactis strains.**

Lanes 1 to 10 contain β-casein incubated with release fractions of L.lactis MG1363 containing respectively plasmids pNZ567 (K138N), pNZ573 (N166D), pNZ521 (wild type), pNZ562 (GDT), pNZ564 (GPP), pNZ563 (GLA), pNZ560 (Δ137-139), pNZ571 (K748T), pNZ565 (ins9) and pNZ566 (ins15).

TABLE B

| Amino acid | single-letter code |
|---|---|
| alanine | A |
| cysteine | C |
| aspartic acid | D |
| glutamic acid | E |
| phenylalanine | F |
| glycine | G |
| histidine | H |
| isoleucine | I |
| lysine | K |
| leucine | L |
| methionine | M |
| asparagine | N |
| proline | P |
| glutamine | Q |
| arginine | R |
| serine | S |
| threonine | T |
| valine | V |
| tryptophan | W |
| tyrosine | Y |

## Claims

1. DNA fragment containing at least one "mutant protease gene", which "mutant protease gene" is understood to mean:
- a gene made up of sections of protease genes of several lactococcal strains, as well as
- a protease gene of a lactococcal strain, the DNA sequence of which has been altered in a manner such that in the protease for which the gene codes:
  (a) an amino acid other than that of the "wild- type" protease is present at at least one site, and/or
  (b) at at least one site within the first 1350 residues of the amino acid sequence, calculated from the N-terminus, at least one amino acid of the "wild- type" protease is missing and/or one or more amino acids have been inserted, or
  (c) at at least two sites separated from each other, one or more amino acids are missing and/or one or more amino acids have been inserted.

2. DNA fragment according to Claim 1, characterized in that said fragment contains at least one protease gene made up of sections of a type I protease gene of L.lactis and a type III protease gene of L.lactis

3. DNA fragment according to Claim 1, characterized in that the fragment contains at least one protease gene made up of sections of protease genes of L.lactis subsp. cremoris Wg2 and L.lactis subsp. cremoris SK11.

4. DNA fragment according to Claim 2 or 3, characterized in that it contains at least one of the hybrid protease genes shown in Fig. 3.

5. DNA fragment according to Claim 1, characterized in that said fragment contains at least one protease gene which is made up of the type I protease gene of L.lactis subsp. cremoris Wg2 to which one or more codons have been added or from which one or more codons are missing and/or have been replaced by an equal or different number of codons.

6. DNA fragment according to one or more of Claims 1-3 and/or 5, characterized in that said fragment contains at least one hybrid protease gene made up of the type I protease gene of L.lactis subsp. cremoris Wg2, of which one or more codons, coding for amino acids as shown in Table A, have been changed into codons which code for the amino acids occurring at the corresponding sites, indicated in Table A, in the type III protease gene of L.lactis subsp. cremoris SK11.

7. DNA fragment according to Claim 6, characterized in that said fragment contains at least one hybrid protease gene made up of the type I protease gene of L.lactis subsp. cremoris Wg2, of which one or more codons coding for amino acids A62, T89, T131, T138, S142, L144, D166, L747 and T748, as shown in Table A, have been changed into codons which code for the amino acids specified in Table A at the corresponding sites in the type III protease gene of L.lactis subsp. cremoris SK11.

8. DNA fragment according to Claim 1 and/or 5, characterized in that said fragment contains at least one protease gene which is made up of the type I protease gene of L.lactis subsp. cremoris Wg2, of which one or more codons coding for amino acids as shown in Table A, in particular the amino acids A62, T89, T131, T138, S142, L144, D166, L747 and/or T748 have been changed into codons which do not code for the amino acids as shown in Table A at corresponding sites in the type III protease gene of L.lactis subsp. cremoris SK11.

9. DNA fragment according to Claim 1 and/or 5, characterized in that said fragment contains at least one protease gene made up of the type I protease gene of L.lactis subsp. cremoris Wg2, of which one or more codons are missing, including at least one codon coding for the amino acids as shown in Table A, in particular the amino acids A62, T89, T131, T138, S142, L144, D166, L747 and T748.

10. DNA fragment according to Claim 1, characterized in that said fragment contains at least one protease gene made up of the type III protease gene of L.lactis subsp. cremoris SK11, to which one or more codons have been added or of which one or more codons are missing and/or have been replaced by an equal or different number of codons.

11. DNA fragment according to one or more of Claims 1-3 and/or 10, characterized in that said fragment contains at least one hybrid protease gene made up of the type III protease gene of L.lactis subsp cremoris SK11, of which one or more codons, coding for amino acids as shown in Table A have been changed into codons which code for amino acids occurring at the corresponding sites, shown in Table A, in the type I protease gene of L. lactis subsp. cremoris Wg2.

12. DNA fragment according to Claim 11, characterized in that said fragment contains at least one hybrid protease gene made up of the type III protease gene of L.lactis subsp. cremoris SK11, of which one or more codons coding for the amino acids V62, K89, S131, K138, A142, V144, N166, R747 and K748 have been changed into codons which code for the amino acids shown in Table A at the corresponding sites in the type I protease gene in L.lactis subsp. cremoris Wg2.

13. DNA fragment according to Claim 1 and/or 10, characterized in that said fragment contains at least one protease gene made up of the type III protease gene of L.lactis subsp. cremoris SK11, of which one or more codons coding for amino acids as shown in Table A, in particular the amino acids V62, K89, S131, K138, A142, V144, N166, R747 and K748 have been changed into codons which do not code for amino acids as shown in Table A at corresponding sites in the type I protease gene of L.lactis subsp. cremoris Wg2.

14. DNA fragment according to Claim 1 and/or 10, characterized in that said fragment contains at least one protease gene made up of the type III protease gene of L.lactis subsp. cremoris SK11, of which one or more codons are missing, including at least one codon coding for the amino acids as shown in Table A, in particular the amino acids V62, K89, S131, K138, A142, V144, N166, R747 and K748.

15. DNA fragment according to Claim 1 and/or 10, characterized in that said fragment contains at least one protease gene made up of type III protease gene of L.lactis subsp. cremoris SK11 of which one or more codons coding for amino acids are missing and/or have been replaced by other codons as illustrated in Fig. 5a and Fig. 5b.

16. DNA fragment according to any one of the claims 1-15, characterized in that said fragment codes for a mutant protease having properties different from the properties of the parent protease(s).

17. Cloning vector containing a DNA fragment according to one or more of Claims 1-16.

18. Vector-according to Claim 17, characterized in that it is a plasmid.

19. Vector according to Claim 17 or 18, characterized in that it can be replicated in a lactococcal host cell.

20. Transformed host strain containing a DNA fragment according to one of Claims 1-16, or a vector according to one of Claims 17-19.

21. Host stain according to Claim 20, characterized in that it is a lactococcal host strain.

22. Host strain according to Claim 21, characterized in that it is a transformed L.lactis strain MG1363.

23. Mutant proteases obtainable with the aid of a host strain according to Claims 20, 21 or 22.

24. Foodstuff produced with at least one host strain according to Claims 20, 21 or 22, or a mutant protease according to Claim 23.

25. Flavouring produced with at least one host strain according to Claims 20, 21 or 22, or a mutant protease according to Claim 23.

# Fig -1 (1)

GCAGTTGCGGTACTTGCCAATAATACTTTAAGGCGCATTTTTTTCTTCATCGATTCGGTCTCCTCTGAAATGCTT -301
A T A T S A L L V K L R M K K K M

ACAGTAAACGTTGTGTATTTATTCTAGCGATGGCAATTGAGAATTTCAATTCAAATACTCATTTTCTAATAATTG -226

CTAAAAATTTCAAAACATCTATAGTCTGTAAACGGCTAAATAATAACGCTAAAAGTTAATTTACAGATAAAAAAA -151

TTAATAGAAGATTAAAATTTTCGTTGAATTTGTTCTTCAATAGTATATAATATAATAGTATATAATATTATATTA -76

TATAATATAATCTTAACTACATCAAGCGTAGGCTTTGATTTGGTTATGAAACTTTTGGAAAGTGGAGGATATTGG -1

ATGCAAAGGAAAAAGAAAGGGCTATCGATCTTGTTAGCCGGTACAGTCGCTTTAGGGGCGCTGGCTGTCTTGCCA 75
M Q R K K K G L S I L L A G T V A L G A L A V L P

GTCGGCGAAATCCAAGCAAAGGCGGCTATCTCGCAGCAAACTAAGGGATCATCACTCGCAAATACGGTGACGGCT 150
V G E I Q A K A A I S Q Q T K G S S L A N T V T A

GCGACTGCTAAGCAAGCGGCCACTGACACAACTGCAGCGACAACGAATCAAGCGATTGCTACACAGTTGGCGGCT 225
A T A K Q A A T D T T A A T T N Q A I A T Q L A A

AAAGGTATTGATTACAATAAGCTGAATAAAGTTCAGCAGCAAGATATTTATGTTGACGTCATTGTTCAAATGAGC 300
K G I D Y N K L N K V Q Q Q D I Y V D V I V Q M S

GCAGCGCCTGCCTCTGAAAACGGCATTTTAAGAACTGATTACTCCAGCACGGCGGAGATTCAGCAGGAGACCAAT 375
A A P A S E N G I L R T D Y S S T A E I Q Q E T N

AAAGTGATCGCGGCTCAGGCAAGCGTTAAAGCAGCTGTTGAACAAGTCACCCAACAAACTGCCGGTGAAAGTTAT 450
K V I A A Q A S V K A A V E Q V T Q Q T A G E S Y

GGCTATGTCGTTAACGGCTTTTCAACTAAAGTTAGGGTTGTTGATATCCCTAAACTGAAACAAATTGCCGGAGTT 525
G Y V V N G F S T K V R V V D I P K L K Q I A G V

AAAACAGTCACATTGGCGAAAGTTTATTATCCGACTGATGCTAAGGCAAACTCGATGGCGAATGTGCAAGCCGTA 600
K T V T L A K V Y Y P T D A K A N S M A N V Q A V 13

TGGTCCAATTACAAATATAAAGGTGAAGGCACAGTTGTCTCGGTTATTGACAGTGGCATTGATCCAACACATAAA 675
W S N Y K Y K G E G T V V S V I D S G I D P T H K 38

CC
GACATGCGGCTAAGCGATGATAAAGACGTTAAACTAACCAAATCTGATGTTGAAAAATTCACTGATACCGTTAAG 750
D M R L S D D K D V K L T K S D V E K F T D T V K 63
A

# fig-1 (2)

```
CATGGCCGCTATTTTAATTCAAAAGTGCCATATGGGTTTAATTACGCTGATAATAACGACACCATTACAGATGAT  825
 H  G  R  Y  F  N  S  K  V  P  Y  G  F  N  Y  A  D  N  N  D  T  I  T  D  D    88

 C
AAGGTTGACGAACAACACGGCATGCATGTTGCTGGGATCATCGGTGCTAACGGGACAGGTGACGATCCAGCCAAG  900
 K  V  D  E  Q  H  G  M  H  V  A  G  I  I  G  A  N  G  T  G  D  D  P  A  K   113
 T
                                                    C                 C
TCTGTTGTCGGAGTTGCACCAGAAGCACAGCTACTGGCAATGAAAGTTTTCAGCAACTCTGACACTTCTGCAAAA  975
 S  V  V  G  V  A  P  E  A  Q  L  L  A  M  K  V  F  S  N  S  D  T  S  A  K   138
                                                    T                 T
          T     T
ACCGGGTCAGCTACCGTGGTTTCTGCCATTGAAGACTCGGCAAAAATCGGTGCCGATGTCCTCAACATGTCCTTA 1050
 T  G  S  A  T  V  V  S  A  I  E  D  S  A  K  I  G· A  D  V  L  N  M  S  L   163
          S     L
       G
GGATCTAATTCAGGCAACCAAACCTTGGAGGATCCAGAACTTGCTGCGGTGCAAAATGCTAACGAATCAGGAACA 1125
 G  S  N  S  G  N  Q  T  L  E  D  P  E  L  A  A  V  Q  N  A  N  E  S  G  T   188
       D
        T
GCCGCCGTCATTTCTGCTGGGAACTCAGGAACATCCGGTTCAGCAACTGAAGGCGTCAACAAAGATTATTACGGT 1200
 A  A  V  I  S  A  G  N  S  G  T  S  G  S  A  T  E  G  V  N  K  D  Y  Y  G   213

                          A
TTGCAAGACAATGAAATGGTGGGATCGCCAGGGACATCACGAGGAGCGACCACAGTTGCTTCCGCTGAAAACACG 1275
 L  Q  D  N  E  M  V  G  S  P  G  T  S  R  G  A  T  T  V  A  S  A  E  N  T   238
                          T

GATGTCATCACTCAGGCAGTGACCATTACAGATGGTACAGGTTTACAGCTTGGACCGGAAACCATTCAGCTTTCA 1350
 D  V  I  T  Q  A  V  T  I  T  D  G  T  G  L  Q  L  G  P  E  T  I  Q  L  S   263

   A                                          G
AGCCACGATTTCACTGGTAGCTTTGACCAAAAGAAGTTTTATATTGTTAAAGATGCTAGTGGCAACCTCAGCAAA 1425
 S  H  D  F  T  G  S  F  D  Q  K  K  F  Y  I  V  K  D  A  S  G  N  L  S  K   288
    N                                        V
  T                                                             C
GGGGCATTAGCCGACTATACTGCTGACGCTAAAGGCAAAATTGCCATCGTTAAACGTGGCGAATTTAGCTTTGAT 1500
 G  A  L  A  D  Y  T  A  D  A  K  G  K  I  A  I  V  K  R  G  E  F  S  F  D   313
                                                              L
                                                       A
GACAAACAAAAATACGCCCAAGCCGCTGGTGCTGCTGGCTTGATCATTGTCAACACCGATGGCACAGCAACACCG 1575
 D  K  Q  K  Y  A  Q  A  A  G  A  A  G  L  I  I  V  N  T  D  G  T  A  T  P   338
                                                       N
 G         G
ATGACTTCTATTGCGTTAACCACCACCTTCCCAACATTTGGGCTCTCCAGTGTAACCGGTCAAAAGCTGGTTGAC 1650
 M  T  S  I  A  L  T  T  T  F  P  T  F  G  L  S  S  V  T  G  Q  K  L  V  D   363
 V        M
                                             G     A  C  G
TGGGTCACAGCACACCCGGATGATAGTCTCGGTGTCAAGATTACCCTGGCGATGTTACCAAATCAGAAATATACT 1725
 W  V  T  A  H  P  D  D  S  L  G  V  K  I  T  L  A  M  L  P  N  Q  K  Y  T   388
                                             A     T  L  V
```

# fig -1 (3)

GAAGACAAGATGTCTGACTTCACATCCTATGGGCCAGTTTCCAATCTTTCCTTCAAACCAGATATTACCGCACCA 1800
E D K M S D F T S Y G P V S N L S F K P D I T A P 413

A
GGCGGCAACATCTGGTCAACGCAAAACAACAATGGCTACACAAATATGTCTGGTACGTCAATGGCCTCGCCATTT 1875
G G N I W S T Q N N N G Y T N M S G T S M A S P F 438

ATTGCCGGTTCACAAGCATTGTTGAAACAAGCATTGAATAACAAAAACAACCCATTTTATGCTTACTACAAACAA 1950
I A G S Q A L L K Q A L N N K N N P F Y A Y Y K Q 463

CTTAAAGGGACAGCGCTCACCGATTTTCTTAAGACAGTTGAGATGAATACTGCCCAGCCAATCAACGATATTAAC 2025
L K G T A L T D F L K T V E M N T A Q P I N D I N 488

T T
TACAATAATGTTATCGTATCGCCGCGGCGGCAAGGGGCCGGTCTGGTTGATGTGAAGGCAGCCATTGATGCATTA 2100
Y N N V I V S P R R Q G A G L V D V K A A I D A L 513

GAAAAGAATCCGTCAACGGTTGTCGCCGAAAACGGCTACCCGGCAGTTGAATTGAAAGACTTCACGAGTACGGAC 2175
E K N P S T V V A E N G Y P A V E L K D F T S T D 538

G A
AAGACCTTTAAACTCACCTTCACGAATCGCACGACCCATGAACTAACCTATCAAATGGACAGTAATACGGATACT 2250
K T F K L T F T N R T T H E L T Y Q M D S N T D T 563
S

AATGCCGTTTATACATCAGCGACTGACCCTAATTCTGGGGTTTTGTATGACAAGAAGATTGATGGAGCAGCCATT 2325
N A V Y T S A T D P N S G V L Y D K K I D G A A I 588

AAAGCTGGCAGTAACATAACTGTGCCTGCTGGGAAAACGGCGCAGATTGAATTCACACTATCTTTGCCGAAGTCT 2400
K A G S N I T V P A G K T A Q I E F T L S L P K S 613

TTTGACCAACAGCAATTTGTTGAAGGTTTTCTGAACTTTAAGGGTAGCGATGGATCGCGCTTGAACTTGCCATAC 2475
F D Q Q Q F V E G F L N F K G S D G S R L N L P Y 638

ATGGGCTTTTTTGGTGACTGGAATGACGGTAAGATTGTCGATAGTCTCAATGGGATCACTTATAGTCCTGCTGGT 2550
M G F F G D W N D G K I V D S L N G I T Y S P A G 663

CC
GGTAATTTTGGCACCGTGCCACTATTGAAAAACAAAAATACAGGCACTCAATATTATGGCGGCATGGTCACAGAC 2625
G N F G T V P L L K N K N T G T Q Y Y G G M V T D 688
T

C
GCTGATGGCAACAAGACAGTTGACGATCAGGCGATTGCTTTTTCGAGTGACAAGAATGCCTTATATAATGACATC 2700
A D G N K T V D D Q A I A F S S D K N A L Y N D I 713
Q

Fig -1(4)

AGCATGAAGTATTATCTATTGCGCAATATCAGCAACGTCCAAGTTGATATTCTTGATGGTCAGGGCAATAAAGTT 2775
S M K Y Y L L R N I S N V Q V D I L D G Q G N K V 738

T C
ACGACTCTCAGCAGTTCCACCAATCGGAAGAAGACCTATTATAATGCTCATTCGCAGCAGTACATCTACTACAAT 2850
T T L S S S T N R K K T Y Y N A H S Q Q Y I Y Y N 763
L T

GCTCCAGCGTGGGATGGCACCTATTATGATCAACGTGATGGCAACATCAAGACGGCTGATGATGGCAGTTATACT 2925
A P A W D G T Y Y D Q R D G N I K T A D D G S Y T 788

TATCGTATTTCCGGTGTACCGGAAGGCGGCGACAAACGTCAAGTGTTTGATGTGCCTTTCAAGCTCGACTCTAAG 3000
Y R I S G V P E G G D K R Q V F D V P F K L D S K 813

GCGCCGACAGTTCGTCATGTCGCTTTGTCAGCCAAAACGGAAAATGGAAAAACCCAGTATTATTTGACAGCTGAA 3075
A P T V R H V A L S A K T E N G K T Q Y Y L T A E 838

C
GCCAAGGATGATTTGAGTGGTCTTGATGCCACCAAGAGCGTTAAAACTGAAATTAATGAAGTGACGAATCTTGAT 3150
A K D D L S G L D A T K S V K T E I N E V T N L D 863
A

GCTACCTTTACCGATGCTGGGACAACGGCTGATGGGTACACCAAAATTGAAACGCCATTATCTGATGAACAGGCC 3225
A T F T D A G T T A D G Y T K I E T P L S D E Q A 888

CAAGCACTTGGCAATGGCGACAATTCGGCTGAGCTGTACTTGACTGATAATGCATCCAATGCCACTGATCAAGAT 3300
Q A L G N G D N S A E L Y L T D N A S N A T D Q D 913

T
GCCAGCGTTCAGAAGCCGGGGTCTACATCGTTTGATTTAATTGTGAACGGCGGCGGTATCCCAGACAAGATCTCA 3375
A S Y Q K P G S T S F D L I V N G G G I P D K I S 938

AGTACCACAACCGGCTACGAAGCCAATACTCAAGGTGGCGGGACGTATACGTTTAGTGGAACGTATCCAGCAGCG 3450
S T T T G Y E A N T Q G G G T Y T F S G T Y P A A 963

A
GTTGACGGTACTTACACTGATGCACAAGGAAAGAAACATGATTTGAACACAACCTACGATGCTGCGACTAACAGT 3525
V D G T Y T D A Q G K K H D L N T T Y D A A T N S 988
N

TTCACTGCCTCAATGCCGGTCACGAATGCTGATTACGCCGCGCAAGTGGATCTATATGCCGATAAGGCGCATACC 3600
F T A S M P V T N A D Y A A Q V D L Y A D K A H T 1013

A C
CAGTTGCTTAAACATTTTGACACCAAAGTTCGGCTGATGGCGCCAACCTTTACTGATTTGAAATTCAACAATGGC 3675
Q L L K H F D T K V R L M A P T F T D L K F N N G 1038
T

fig-1(5)

TCGGATCAGACCTCTGAAGCGACCATCAAGGTTACAGGGACGGTTAGTGCTGACACCAAGACAGTTAATGTTGGC 3750
S D Q T S E A T I K V T G T V S A D T K T V N V G 1063

G
CACACCGTAGCAGCACTTGATGCACAACATCACTTTAGTGTTGATGTACCGGTTAATTATGGTGACAATACCATC 3825
H T V A A L D A Q H H F S V D V P V N Y G D N T I 1088
D

G C
AAGGTGACCGCCACCGACAAAGATGGCAACACCACGACGGAGCAAAAGACGATTACCTCGTCTTATGATCCTGAT 3900
K V T A T D K D G N T T T E Q K T I T S S Y D P D 1113
E

T C G G
ATGTTGAAGAAGTCTGTGACGTTCGATCAAGGTGTGAAATTTGGTACCAATAAATTCAATGCCACCTCGGCTAAG 3975
M L K K S V T F D Q G V K F G T N K F N A T S A K 1138
N T A E

TTCTATGACCCTAAGACCGGGATTGCGACGATTACTGGTAAGGTCAAGCACCCAACGACAACGTTGCAGGTTGAT 4050
F Y D P K T G I A T I T G K V K H P T T T L Q V D 1163

GGTAAGCAAATTCCAATCAAGGATGATCTGACTTTCAGTTTCACTTTAGATTTAGGTACTCTTGGACAAAAACCG 4125
G K Q I P I K D D L T F S F T L D L G T L G Q K P 1188

G A
TTTGGGGTTGTTGTGGGTGACACCACTCAAAACAAAACCTTCCAAGAAGCGTTGTCCTTCATTTTGGATGCAGTG 4200
F G V V V G D T T Q N K T F Q E A L S F I L D A V 1213
T

G
GCTCCAACATTGTCATTGGATAGCTCGACAGATGCACCGGTTTATACCAACGATCCAAACTTCCAGATTACCGGA 4275
A P T L S L D S S T D A P V Y T N D P N F Q I T G 1238
E

T
ACGGCCACTGACAATGCGCAATATCTGAGTCTGTCAATTAACGGCAGTTCTGTCGCCAGCCAATACGAAGACATC 4350
T A T D N A Q Y L S L S I N G S S V A S Q Y E D I 1263
V

T
AACATCAATAGCGGCAAACCAGGTCATATGGCTATTGATCAGCCCGTTAAATTGCTCGAAGGCAAAAACGTGCTG 4425
N I N S G K P G H M A I D Q P V K L L E G K N V L 1288

ACTGTTGCTGTTACAGATAGCGAAGACAACACCACGACCAAGAACATCACAGTTTACTACGAACCAAAGAAAACA 4500
T V A V T D S E D N T T T K N I T V Y Y E P K K T 1313

A. T
CTGGCAGCACCAACTGTGACGCCAAGTACCACTGAACCAGCCCAAACGGTGACTCTGACGGCAAACGCTGCCGCA 4575
L A A P T V T P S T T E P A Q T V T L T A N A A A 1338
K S

G
ACGGGCGAAACGGTTCAGTATAGTGCTGATGGTGGCAAGACATATCAGGATGTTCCGGCAGCCGGTGTCACGATC 4650
T G E T V Q Y S A D G G K T Y Q D V P A A G V T I 1363
V

Fig-1(6)

ACGGCAAATGGCACCTTCAAGTTTAAGTCGACTGATTTATACGGTAATGAATCACCAGCCGTCGACTATGTTGTC 4725
T A N G T F K F K S T D L Y G N E S P A V D Y V V 1388

ACCAATATCAAGGCCGATGATCCTGCACAATTGCAGGCAGCTAAGCAGGAACTGACTAATCTGATTGCTTCCGCC 4800
T N I K A D D P A Q L Q A A K Q E L T N L I A S A 1413

AAAACGCTAAGTGCCAGCGGTAAGTATGATGATGCCACAACGACTGCTTTAGCAGCGGCAACGCAGAAGGCACAA 4875
K T L S A S G K Y D D A T T T A L A A A T Q K A Q 1438

ACGGCGCTTGATCAGACGAACGCCTCAGTTGATTCACTTACTGGTGCCAATCGAGACCTGCAAACTGCGATCAAT 4950
T A L D Q T N A S V D S L T G A N R D L Q T A I N 1463

C
CAATTAGCTGCCAAGTTACCTGCAGATAAGAAGACTTCGCTGCTTAACCAGTTGCAATCTGTGAAGGATGCGCTG 5025
Q L A A K L P A D K K T S L L N Q L Q S V K D A L 1488
A

GGAACGGACTTGGGCAATCAAACTGATCCAAGCACTGGCAAAACATTTACGGCAGCGTTAGACGACCTAGTGGCA 5100
G T D L G N Q T D P S T G K T F T A A L D D L V A 1513

A G G C
CAAGCTCAAGCAGGCACGCAAACGGACGACCAGCTTCAAGCAACTCTTGCCAAGATACTTGATGAAGTATTAGCA 5175
Q A Q A G T Q T D D Q L Q A T L A K I L D E V L A 1538
H . V A

AAACTTGCGGAGGGTATTAAAGCGGCAACACCGGCTGAGGTTGGCAATGCTAAAGATGCTGCAACTGGCAAAACT 5250
K L A E G I K A A T P A E V G N A K D A A T G K T 1563

TGGTATGCCGACATTGCTGACACATTGACGTCTGGTCAAGCCAGTGCTGATGCGTCTGACAAGCTTGCACATTTA 5325
W Y A D I A D T L T S G Q A S A D A S D K L A H L 1588

CAAGCTTTGCAAAGTCTGAAAACGAAGGTGGCAGCTGCCGTTGAAGCGGCCAAGACAGTTGGTAAAGGCGACGGT 5400
Q A L Q S L K T K V A A A V E A A K T V G K G D G 1613

ACAACCGGTACTAGCGACAAAGGCGGCGGTCAAGGTACCCCGGCGCCCGCTCCAGGCGACACAGGTAAGGACAAA 5475
T T G T S D K G G G Q G T P A P A P G D T G K D K 1638

GGAGATGAGGGCAGCCAGCCTAGTTCTGGCGGTAATATCCCAACAAAGCCAGCCACAACGACGTCAACGACCACG 5550
G D E G S Q P S S G G N I P T K P A T T T S T T T 1663

GATGATACGACTGATCGTAATGGTCAACTTACATCCGGTACTAGCGACAAAGGCGGCGGTCAAGGTACCCCGGCG 5625
D D T T D R N G Q L T S G T S D K G G G Q G T P A 1688

fig-1 (7)

```
CCCGCTCCAGGCGACATAGGTAAGGACAAAGGCGATGAGGGCAGCCAGCCTAGTTCTGGCGGTAATATCCCAACA 5700
 P  A  P  G  D  I  G  K  D  K  G  D  E  G  S  Q  P  S  S  G  G  N  I  P  T  1713

G
AATCCAGCCACAACGACGTCAACGACCACGGATGATACGACTGATCGTAATGGTCAACTTACATCCGGTAAGGGA 5775
 N  P  A  T  T  T  S  T  T  T  D  D  T  T  D  R  N  G  Q  L  T  S  G  K  G  1738
                            S

                                                                     T
GCATTACCCAAGACAGGAGAGACAACTGAGCGGCCAGCGTTTGGCTTCTTGGGTGTCATTGTGGTCAGTCTGATG 5850
 A  L  P  K  T  G  E  T  T  E  R  P  A  F  G  F  L  G  V  I  V  V  S  L  M  1763
                                                                     I

GGGGTATTAGGATTGAAACGGAAACAACGTGAAGAATAGCCTGTCCATGCGTTGCGTTTAGGGCAGCAGCGTATC 5925
 G  V  L  G  L  K  R  K  Q  R  E  E  *                                      1775

GACAAAAAGAAAAGGGCGCTACGATATTTGGAGTTGAGGTTCAAAGTCAAATGGTACTGATGACCGGTAAAATTT 6000

AATATTTTGAACCTTGCTTAGGCAGCTGACTTCACATTGTTGAGATCAGCTGCCTTTTGCTTATAGTTCATTGAG 6075

TAGAAACGGTTCTGTTGCGAAGTTTGAAAATCAAACGCGCCCGCTCTGAACTCCAAATATCTTAGGCTGTTATTC 6150

CCATTAATACCTTGATTTCAGTAGACACCGAAAAGCCGAAGAGCGTTCCATTTCTTCGGTTATTTTTATATATTC 6225

CTCGAAGGGTCTCCATGCCCTTAATCGTGGAAGAGGCTGTACGGAGACTTTGATAAAATTTAATCCGTCGTTTAA 6300
```

Fig-2

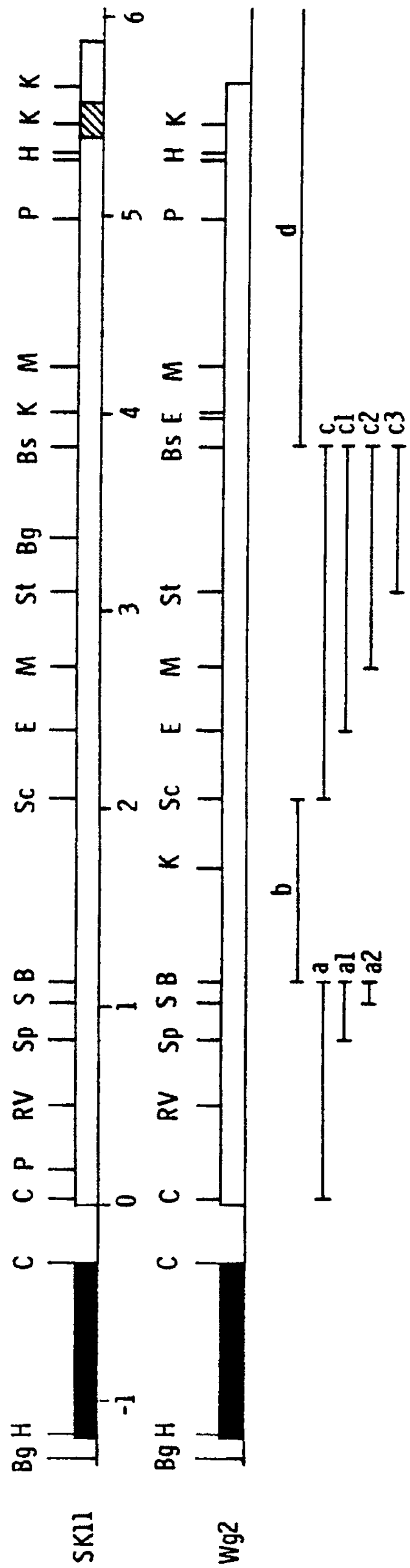
SK11
Wg2
Bg H
C
C P
RV
Sp S B
Sc
E
M
St
Bg
Bs K
M
P
H K K
K
Bs E
H K
-1
0
1
2
3
4
5
6
a
a1
a2
b
c
c1
c2
c3
d

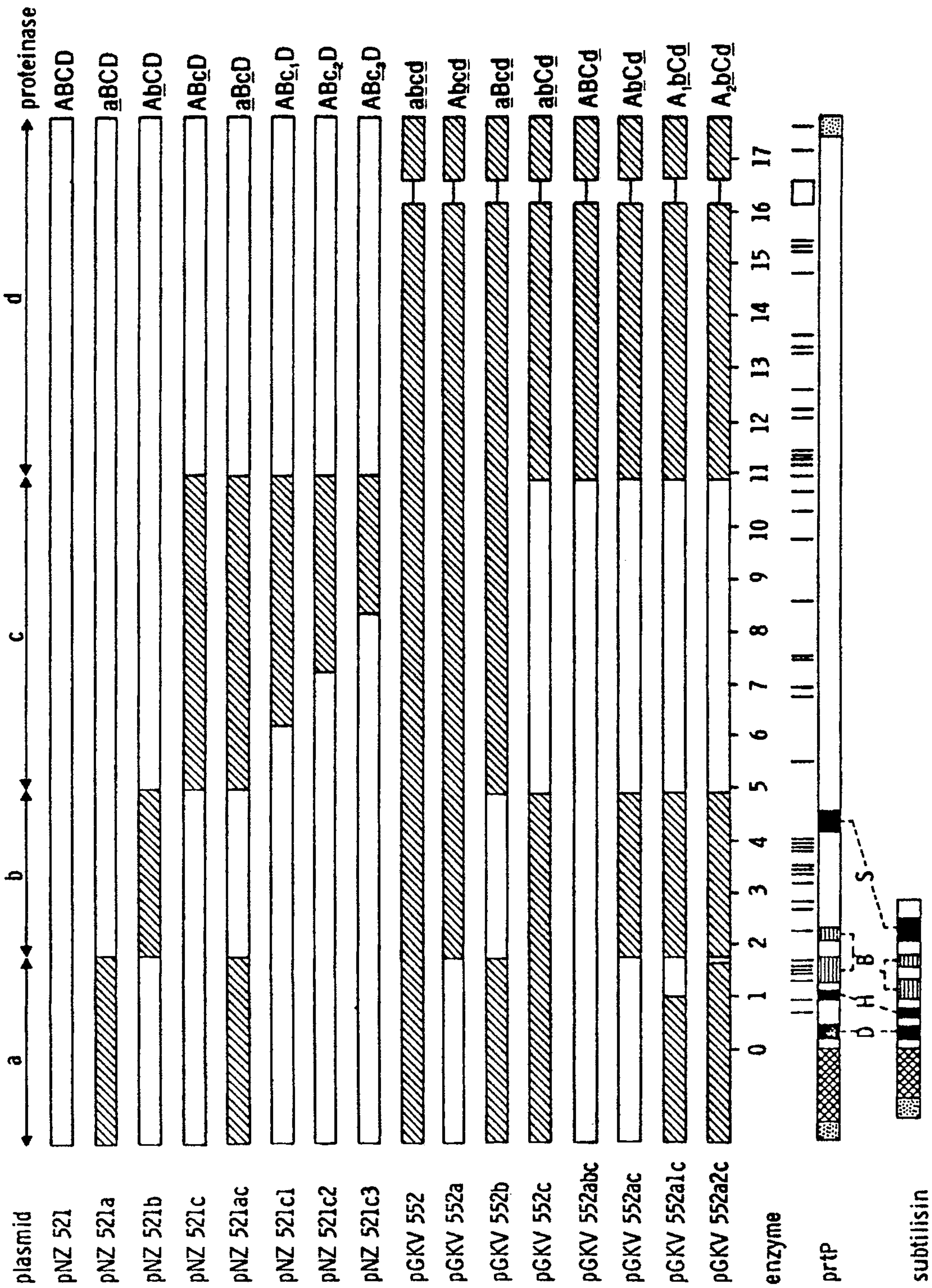
plasmid
pNZ 521
pNZ 521a
pNZ 521b
pNZ 521c
pNZ 521ac
pNZ 521c1
pNZ 521c2
pNZ 521c3
pGKV 552
pGKV 552a
pGKV 552b
pGKV 552c
pGKV 552abc
pGKV 552ac
pGKV 552a1c
pGKV 552a2c
enzyme
prtP
subtilisin
a
b
c
d
proteinase
ABCD
aBCD
AbCD
ABcD
abcD
ABc1D
ABc2D
ABc3D
abcd
Abcd
aBcd
abCd
ABCd
AbCd
A1bCd
A2bCd
0 1 2 3 4 5 6 7 8 9 10 11 12 13 14 15 16 17
D H B S

Fig-3

Fig-4a

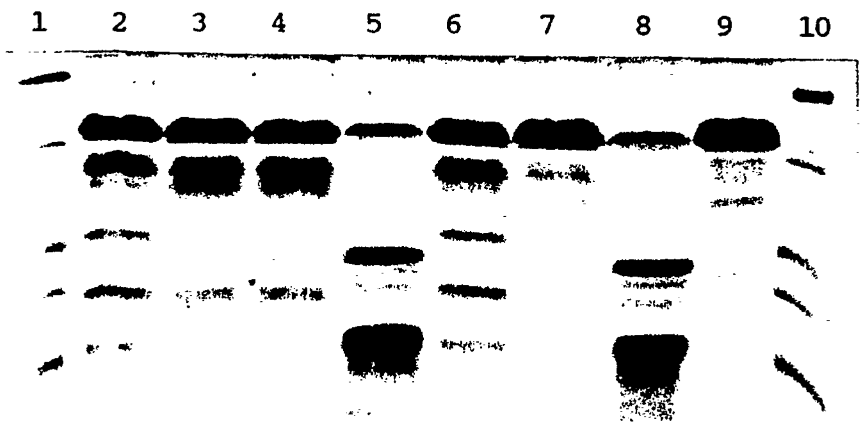


Fig-4b

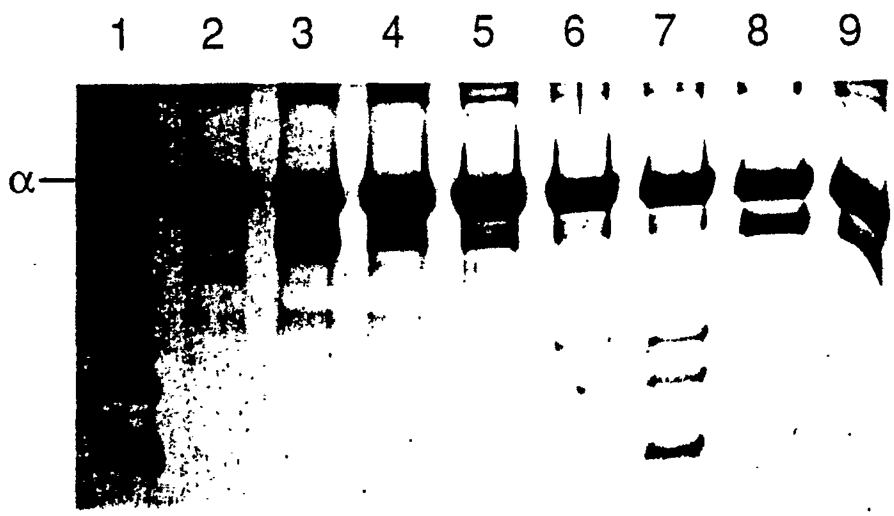

fig-4c

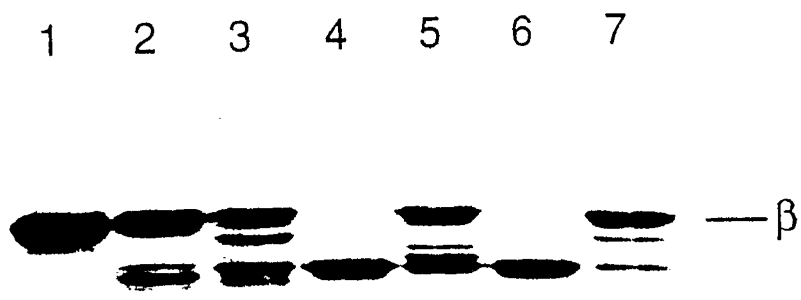


fig-4d

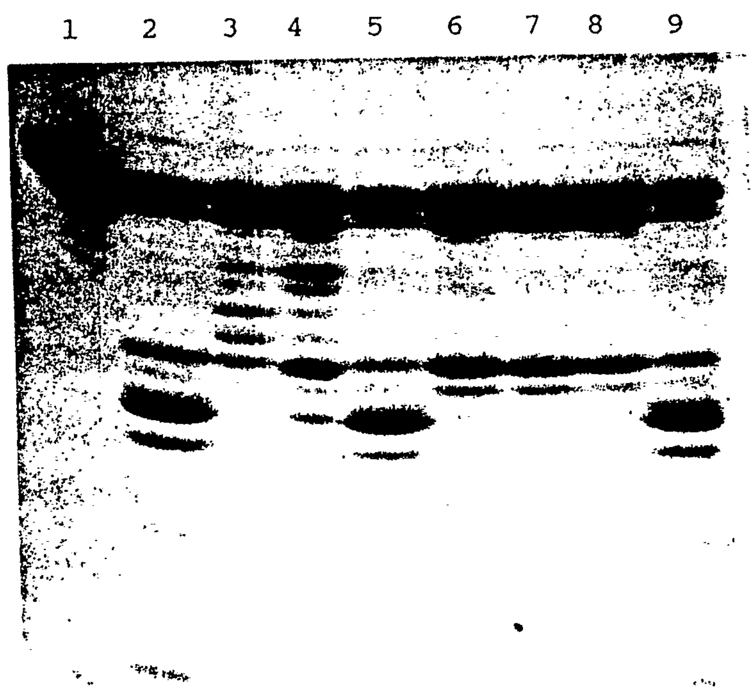

fig-5a

```
               430                                 440
          T  N  M  S  G  T  S  M  A  S  P  F  I  A
SK11:     ACAAATATGTCTGGTACGTCAATGGCCTCGCCATTTATTGCC
                                 *
S433A:               5-TCTGGTACGGCAATGGCCTC-3
                        S  G  T  A  M  A  S


       160                           170
          N  M  S  L  G  S  N  S  G  N  Q  T  L  E
SK11:     AACATGTCCTTAGGATCTAATTCAGGCAACCAAACCTTGGAG
                                 *
N166D:              5-CTTAGGATCTGATTCAGGCA-3
                        L  G  S  D  S  G


                                         750
              L  S  S  S  T  N  R  K  K  T  Y  Y  N  A
SK11:         CTCAGCAGTTCCACCAATCGGAAGAAGACCTATTATAATGCT
                                    *
R747L:                   5-CCACCAATCTGAAGAAGACCTAT-3
                           S  T  N  L  K  K  T  Y

                                       *
K748T:                   5-CCACCAATCGGACGAAGACCTAT-3
                           S  T  N  R  T  K  T  Y


                                   140
          N  S  D  T  S  A  K  T  G  S  A  T  V  V
SK11:     AACTCTGACACTTCTGCAAAAACCGGGTCAGCTACCGTGGTT
                                *
K138N:                5-CTTCTGCAAACACCGGGT-3


                                         1760
                      F  L  G  V  I  V  V  S  L  M
SK11:                 TTCTTGGGTGTCATTGTGGTCAGTCTGATG
                                         **
V1759R:                   5-GGTGTCATTAGGGTCAGTCTG-3
                             G  V  I  R  V  S  L
```

fig-5b

```
                                      140
              N  S  D  T  S  A  K  T  G  S  A  T  V  V
SK11:         AACTCTGACACTTCTGCAAAAACCGGGTCAGCTACCGTGGTT
                            *           *
Δ137-139:      5-TCTGACACTTCC ———————— GGATCAGCTACCG-3

                                    140
         D  T       S  G  D        T  G  S  A
GDT :    GACACTT    CCGGGGACA      CCGGATCAGCT
         CTGTGAAGGCC    CCTGTGGCC      TAGTCGA

                                    140
         D  T       S  G  L        A  G  S  A
GLA :    GACACTT    CCGGTCTTG      CCGGATCAGCT
         CTGTGAAGGCC    AGAACGGCC      TAGTCGA

                                    140
         D  T       S  G  P        P  G  S  A
GPP :    GACACTT    CCGGTCCCC      CCGGATCAGCT
         CTGTGAAGGCC    AGGGGGGCC      TAGTCGA

                                                     140
         D  T      S  G  S  A  G  T  T  G  T      T  G  S  A
INS9:    GACACTT   CCGGTAGTGCCGGCACTACCGGCACTA    CCGGATCAGCT
         CTGTGAAGGCC   ATCACGGCCGTGATGGAAGTGATGGCC    TAGTCGA

                                                                         140
         D  T      S  G  K  T  G  L  A  G  K  T  G  K  T  G  K      T  G  S  A
INS15:   GACACTT   CCGGCAAGACCGGTCTTGCCGGCAAGACCGGCAAGACCGGCAACA    CCGGATCAGCT
         CTGTGAAGGCC   GTTCTGGCCAGAACGGCCGTTCTGGCCGTTCTGGCCGTTGTGGCC    TAGTCGA

Δ205-219/S:

         200                        210                         220
         T  S  G  S  A  T  E  G  V  N  K  D  Y  Y  G  L  Q  D  N  E  M  V  G  S  P  G
SK11:    ACATCCGGTTCAGCAACTGAAGGCGTCAACAAAGATTATTACGGTTTGCAAGACAATGAAATGGTGGGATCGCCAGGG

          5-TCCGGTTCAGCAACT ——————— AGT ———————— GTGGGATCGCC-3
```

fig-6